(19) Europäisches Patentamt
European Patent Office
Office européen des brevets

(11) Veröffentlichungsnummer: **0 084 164**
**B1**

(12) **EUROPÄISCHE PATENTSCHRIFT**

(45) Veröffentlichungstag der Patentschrift: **28.01.87**

(51) Int. Cl.⁴: **C 07 D 209/42**, C 07 D 209/52, A 61 K 31/40

(21) Anmeldenummer: **82112007.8**

(22) Anmeldetag: **24.12.82**

(54) **Neue Derivate bicyclischer Aminosäuren, Verfahren zu ihrer Herstellung, diese enthaltende Mittel und deren Verwendung sowie neue bicyclische Aminosäuren als Zwischenstufen und Verfahren zu deren Herstellung.**

(30) Priorität: **29.12.81 DE 3151690**
**24.03.82 DE 3210701**

(43) Veröffentlichungstag der Anmeldung: **27.07.83 Patentblatt 83/30**

(45) Bekanntmachung des Hinweises auf die Patenterteilung: **28.01.87 Patentblatt 87/5**

(84) Benannte Vertragsstaaten: **AT BE CH DE FR GB IT LI LU NL SE**

(56) Entgegenhaltungen:
**EP - A - 0 031 741**
**EP - A - 0 037 231**
**EP - A - 0 051 301**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Urbach, Hansjörg, Dr., Le Lavandoustrasse 41, D-6242 Kronberg/Taunus (DE)**
Erfinder: **Henning, Rainer, Dr., Völklinger Weg 56, D-6000 Frankfurt am Main 71 (DE)**
Erfinder: **Teetz, Volker, Dr., An der Tann 20, D-6238 Hofheim am Taunus (DE)**
Erfinder: **Geiger, Rolf, Prof. Dr., Heinrich-Bleicher-Strasse 33, D-6000 Frankfurt am Main 50 (DE)**
Erfinder: **Becker, Reinhard, Dr., Adelheidstrasse 101, D-6200 Wiesbaden (DE)**
Erfinder: **Gaul, Holger, Albert-Schweitzer-Strasse 2, D-6251 Niederneisen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Aus der EP-A-0 037 231 sind substituierte Acylderivate von cis, endo-Octahydro-1H-indol-2-carbonsäure und ihre Verwendung als Hemmer des Angiotensin-Converting-Enzyms (ACE) bekannt.

Es wurde nun gefunden, dass bestimmte Derivate transkonfigurierter bicyclischer Aminosäuren eine starke ACE-hemmende Wirkung aufweisen.

Die vorliegende Erfindung betrifft daher neue Derivate bicyclischer Aminosäuren der Formel I,

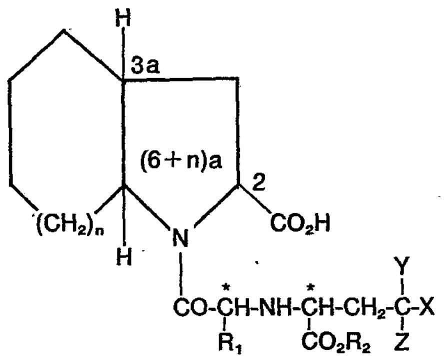
I

in der die Wasserstoffatome an den Brückenkopf-C-Atomen 3a und (6+n)a zueinander trans-konfiguriert sind, und worin

$n = 0$, 1 oder 2,
$r_1 =$ Methyl,
$R_2 =$ Wasserstoff, $(C_1–C_4)$-Alkyl oder Benzyl,
Y = Wasserstoff oder Hydroxy,
Z = Wasserstoff oder
Y und Z = zusammen Sauerstoff und
X = Phenyl bedeuten,

sowie deren physiologisch unbedenklichen Salze.

Als Salze kommen insbesondere infrage Alkali- und Erdalkalisalze, Salze mit physiologisch verträglichen Aminen und Salze mit anorganischen oder organischen Säuren wie z.B. HCl, HBr, $H_2SO_4$, Maleinsäure, Fumarsäure.

Alkyl kann geradkettig oder verzweigt sein.

Im hier vorliegenden Fall der trans-Konfiguration der H-Atome an C-3a und C-(6+n)a kommen zwei mögliche Konfigurationen des Bicyclus in Betracht, und zwar die 2β, 3aα, (6+n)aβ-Konfiguration (Formelrest Ib) sowie die 2β, 3aβ, (6+n)aα-Konfiguration (Formelrest Ic).

(Zur Definition von α und β s.a. Fieser, Steroide, S. 2, 1961).

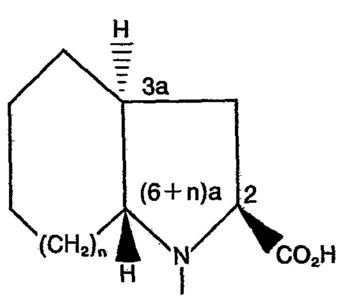
Ib

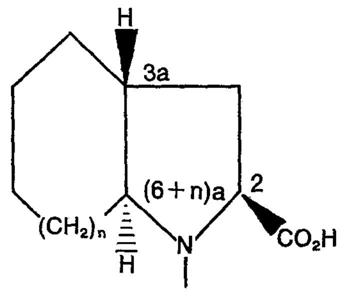
Ic

Verbindungen der Formel I besitzen chirale C-Atome in den Positionen C-2, C-3a, C-(6+n)a, sowie in den mit einem Stern markierten C-Atomen der Seitenkette. Sowohl die R- als auch die S-Konfigurationen an allen Zentren sind Gegenstand der Erfindung. Die Verbindungen der Formel I können daher als optische Isomere, als Diastereomere, als Racemate oder als Gemische derselben vorliegen. Bevorzugt sind die Verbindungen der Formel I, in denen das C-Atom 2 im bicyclischen Ringsystem, sowie die mit einem Stern markierten C-Atome der Seitenkette S-Konfiguration aufweisen.

Bevorzugt sind solche Verbindungen der Formel I, in der $R_2 =$ Wasserstoff oder Ethyl bedeuten.

Von den Verbindungen der Formel I sind
N-(1S-Carboxy-3-phenyl-propyl)-S-alanyl-2S,3aR,7aS-octahydroindol-2-carbonsäure und
N-(1S-Carboxy-3-phenyl-propyl)-S-alanyl-2S,3aS,7aR-octahydroindol-2-carbonsäure
insbesondere aber
N-(1S-Carbethoxy-3-phenyl-propyl)-S-alanyl-2S,3aR,7aS-octahydroindol-2-carbonsäure, und
N-(1S-Carbethoxy-3-phenyl-propyl)-S-alanyl-2S,3aS,7aR-octahydroindol-2-carbonsäure
hervorzuheben.

Die Erfindung betrifft ferner ein Verfahren zur Herstellung der Verbindungen der Formel I.

Eine Verfahrensvariante ist dadurch gekennzeichnet, dass man eine Verbindung der Formel II,

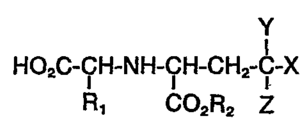
II

worin $R_1$, $R_2$, X, Y und Z die Bedeutungen wie in Formel I haben, mit einer Verbindung der Formel III,

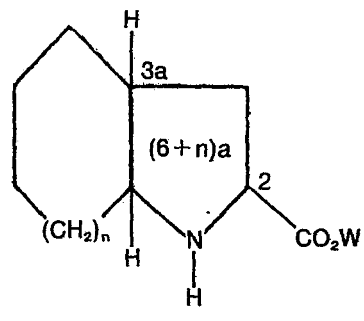
III

in der die H-Atome an den C-Atomen 3a und (6+n)a zueinander trans-konfiguriert sind, und worin

n = 0, 1 oder 2 und

W = einen hydrogenolytisch oder sauer abspaltbaren Rest, insbesondere einen Benzyl- oder einen tert.-Butyl-Rest bedeuten,

nach bekannten Amidbildungsmethoden der Peptidchemie umsetzt und anschliessend durch katalytische Hydrierung oder Säure-Behandlung den Rest W und gegebenenfalls durch zusätzliche Säure- oder Basenbehandlung auch den Rest $R_2$ abspaltet, wobei jeweils die freien Carbonsäuren erhalten werden.

Weitere Syntheseverfahren zur Herstellung der Verbindungen der Formel I, in der Y und Z zusammen Sauerstoff bedeuten, bestehen darin, dass man eine Verbindung der Formel IV,

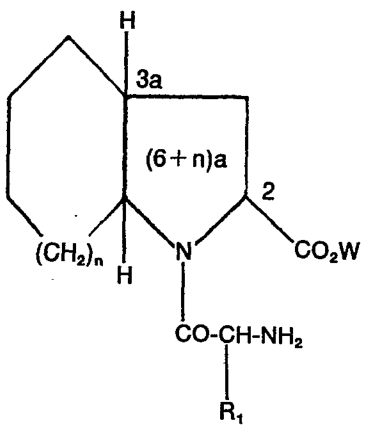

IV

worin die H-Atome an den C-Atomen 3a und (6+n)a zueinander trans-konfiguriert sind, und worin n und $R_1$ die Bedeutungen wie in Formel I, besitzen und W die Bedeutung wie in Formel III hat, mit einer Verbindung der Formel V

$$R_2O_2C\text{-}CH=CH\text{-}CO\text{-}X \qquad V$$

worin $R_2$ und X die Bedeutungen wie in Formel I besitzen in bekannter Weise in einer Michael-Reaktion (Organikum, 6. Auflage, S. 492, 1967) umsetzt und den Rest W und gegebenenfalls den Rest $R_2$ wie oben beschrieben abspaltet oder dass man eine Verbindung der obengenannten Formel IV mit einer Verbindung der allgemeinen Formel VI, worin $R_2$ die Bedeutung wie in Formel I hat, und mit einer Verbindung der allgemeinen Formel VII

$OHC\text{–}CO_2R_2$ (VI) $\qquad$ $X\text{–}CO\text{–}CH_3$ (VII)

worin X die Bedeutung wie in Formel I hat, in bekannter Weise in einer Mannich-Reaktion (Bull. Soc. Chim. France 1973, S. 625) umsetzt und anschliessend den Rest W und gegebenenfalls den Rest $R_2$ wie oben beschrieben unter Bildung der freien Carboxygruppen abspaltet.

Ferner können Verbindungen der Formel I mit Y, Z jeweils = Wasserstoff auch in der Weise hergestellt werden, dass man eine Verbindung der obengenannten Formel IV gemäss der in J. Amer. Chem. Soc. 93 2897 (1971) beschriebenen Verfahrensweise mit einer Verbindung der Formel VIII

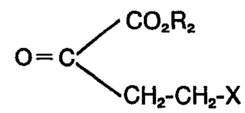

VIII

worin $R_2$ und X die Bedeutungen wie in Formel I besitzen, umsetzt, die erhaltenen Schiff-Basen reduziert und anschliessend den Rest W und gegebenenfalls den Rest $R_2$ wie oben beschrieben, unter Bildung der freien Carboxygruppen abspaltet, oder dass man eine gemäss obigen Verfahrensweisen erhaltene Verbindung der Formel I, in der Y und Z zusammen Sauerstoff bedeuten, katalytisch mit Wasserstoff reduziert. Die Reduktion der Schiff-Basen kann katalytisch, elektrolytisch oder mit Reduktionsmitteln wie beispielsweise Natriumborhydrid oder Natriumcyanborhydrid erfolgen.

Verbindungen der Formel I mit Y = Hydroxy und Z = Wasserstoff können beispielsweise auch durch Reduktion einer gemäss obigen Verfahrensweisen erhaltenen Verbindung I mit Y und Z = zusammen Sauerstoff erhalten werden. Diese Reduktion kann katalytisch mit Wasserstoff oder mit einem anderen Reduktionsmittel wie beispielsweise Natriumborhydrid erfolgen.

Die nach oben genannter Verfahrensweise erhaltenen Verbindungen der Formel I werden gegebenenfalls in ihre physiologisch verträglichen Salze überführt.

Die Erfindung betrifft weiterhin Verbindungen der Formel III'

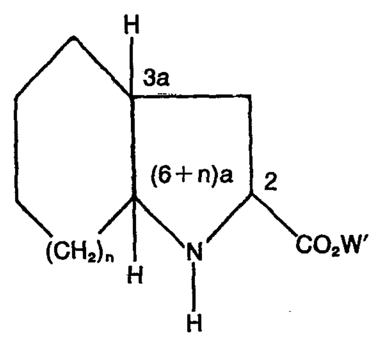

III'

in der die H-Atome an den C-Atomen 3a und (6+n)a zueinander trans-konfiguriert sind und worin

n = 0, 1 oder 2

W' = Wasserstoff, $(C_1\text{–}C_{18})$-Alkyl oder $(C_7\text{–}C_{10})$-Aralkyl bedeuten.

Diese Verbindungen dienen gemäss der Erfindung als Ausgangsstoffe bei der Synthese von Verbindungen der Formel I und können erfindungsgemäss nach folgenden Verfahrensweisen hergestellt werden.

Bei einer Synthesevariante geht man von einer Verbindung der Formel IX aus,

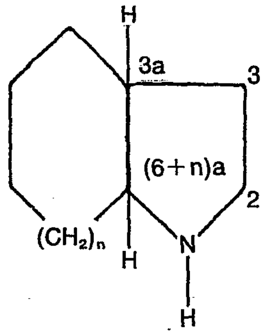

IX

worin die H-Atome an den C-Atomen 3a und (6+n)a zueinander trans-konfiguriert sind und worin n die Zahl 0, 1 oder 2 bedeutet.

Verbindungen der Formel IX mit n = 0 sind aus Booth et al., J. Chem. Soc. 1959 S. 1050, solche mit n = 1 aus King et al., J. Chem. Soc. 1953, S. 250, 253 und solche mit n = 2 aus Ayerst et al., J. Chem. Soc. 1960, S. 3445 bekannt.

Diese Verbindungen der Formel IX werden in bekannter Weise acyliert, wobei ein aliphatischer oder aromatischer Acyl-Rest, vorzugsweise ein Acetyl- oder Benzoyl-Rest an das Stickstoffatom gebunden wird, und die erhaltenen N-acylierten Verbindungen in einem aliphatischen Alkohol, bevorzugt einem Alkanol mit 1 bis 4 C-Atomen, insbesondere Methanol, in Gegenwart eines Leitsalzes vorzugsweise bei Temperaturen im Bereich von 0° bis +40 °C unter Bildung einer Verbindung der Formel X, worin n = 0, 1, 2 und

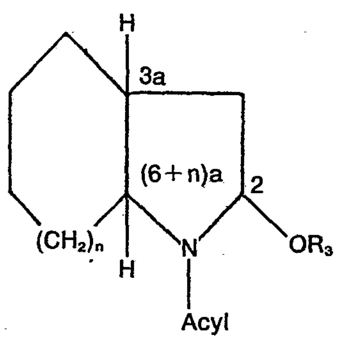

X

$R_3$ = ($C_1$–$C_4$)-Alkyl bedeuten, anodisch oxidiert (analog: Liebigs Ann. Chem. 1978, S. 1719).

Die erhaltene Verbindung der allgemeinen Formel X wird mit Trimethylsilylcyanid nach Tetrahedron Letters 1981, S. 141 in einem aprotischen organischen Lösemittel wie beispielsweise in einem Kohlenwasserstoff, Halogenkohlenwasserstoff, in Äther oder in THF bei Temperaturen im Bereich von −60 °C bis +20 °C, vorzugsweise −40 °C bis ±0 °C in Gegenwart einer Lewis-Säure wie beispielsweise $ZnCl_2$, $SnCl_2$, $SnCl_4$, $TiCl_4$, $BF_3$-Ätherat, vorzugsweise $BF_3$-Ätherat, umgesetzt und die erhaltene Verbindung der Formel XI,

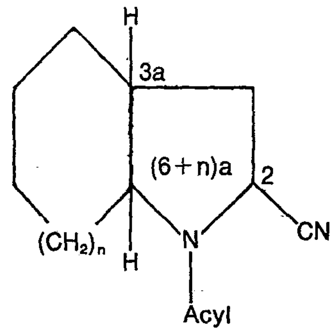

XI

worin die H-Atome an den C-Atomen 3a und (6+n)a zueinander trans-konfiguriert sind und worin n die oben genannte Bedeutung hat, nach Reinigung und Abtrennung von Nebenprodukten mittels Umkristallisation oder Säulenchromatographie durch Einwirkung von Säuren oder Basen in bekannter Weise zu einer Verbindung der Formel III′ mit W′ = Wasserstoff hydrolysiert und letztere gegebenenfalls verestert. Bei der sauren Hydrolyse der Nitrilgruppe wird insbesondere HCL oder HBr als Säure verwendet. Die Veresterung wird hier wie im folgenden gemäss den in der Aminosäurenchemie üblichen Verfahrensweisen durchgeführt.

Verbindungen der allgemeinen Formel III′ können auch hergestellt werden, indem man eine Verbindung der Formel XII

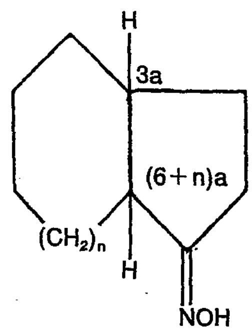

XII

worin die H-Atome an den C-Atomen 3a und (6+n)a trans-konfiguriert sind und n die oben genannte Bedeutung besitzt in einer Beckmann-Umlagerung analog Helv. Chim. Acta 46, 1190, (1963) zu einer Verbindung der Formel XIII, worin n die obengenannte Bedeutung besitzt,

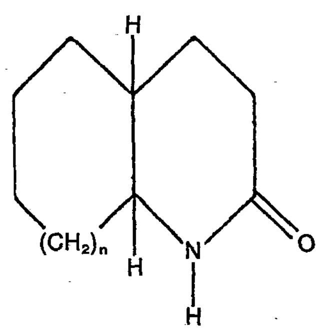

XIII

umsetzt und diese zu einer Verbindung der Formel XIV,

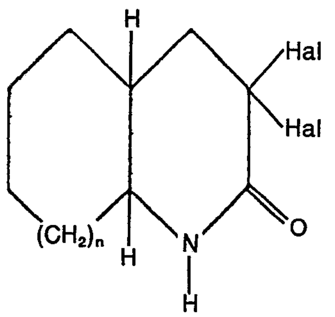

XIV

worin n die obengenannte Bedeutung besitzt und Hal ein Halogenatom, vorzugsweise Chlor oder Brom bedeutet, halogeniert. Als Halogenierungsmittel kommen beispielsweise anorganische Säurehalogenide wie $PCl_5$, $SO_2Cl_2$, $POCl_3$, $SOCl_2$, $PBr_3$ oder Halogene wie Brom in Betracht. Von Vorteil ist die Verwendung von $PCl_5$ oder $POCl_3$ in Kombination mit $SO_2Cl_2$. Als Zwischenstufe bildet sich zunächst ein Imidhalogenid, das mit den genannten Halogenierungsmitteln und anschliessender Hydrolyse unter basischen Bedingungen, vorzugsweise mit wässrigem Alkalicarbonat weiter zu einer Verbindung der Formel XIV reagiert.

Die Verbindungen der Formel XIV werden nachfolgend in einem polaren protischen Lösungsmittel wie beispielsweise einem Alkohol, vorzugsweise Ethanol, oder einer Carbonsäure wie beispielsweise Essigsäure unter Zusatz eines Säureacceptors wie beispielsweise Natriumacetat oder Triethylamin zu einer Verbindung der Formel XV,

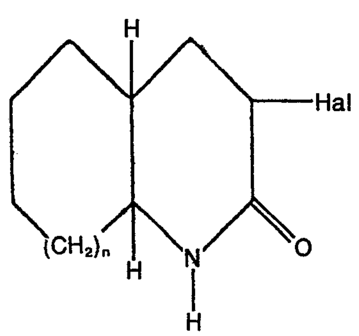

XV

worin n und Hal die oben genannten Bedeutungen besitzen, katalytisch reduziert. Als Katalysator kommen beispielsweise Raney-Nickel oder Palladium bzw. Platin auf Tierkohle in Betracht.

Verbindungen der Formel XV können auch direkt durch Halogenierung der Verbindungen der Formel XIII beim Einsatz geringerer Mengen der obengenannten Halogenierungsmittel hergestellt werden.

Verbindungen der Formel XV werden gemäss der bekannten Favorskii-Reaktion in Gegenwart einer Base zu einer Verbindung der Formel III′ mit W′ = Wasserstoff umgesetzt und diese gegebenenfalls verestert. Die obengenannte Favorskii-Reaktion wird in einem alkoholischen Lösungsmittel wie Methanol, Ethanol oder tert.-Butanol oder in Wasser oder in Gemischen derselben bei Temperaturen im Bereich von 20° bis 140 °C, vorzugsweise zwischen 60° und 100 °C durchgeführt. Als Basen werden zweckmässig Alkali- oder Erdalkalihydroxide wie Natrium-, Kalium- oder Bariumhydroxid oder Alkalialkoholate wie beispielsweise Natriummethylat oder Kalium-tert.-butanolat eingesetzt.

Verbindungen der Formel III′, in der die H-Atome an den C-Atomen 3a und 6+n)a trans-Konfiguration aufweisen können auch aus einer Verbindung der Formel XVI

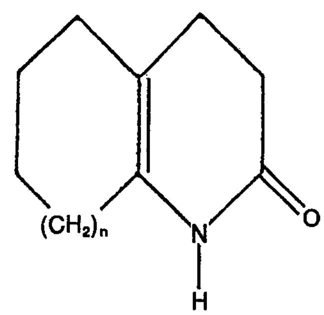

XVI

worin n die oben genannte Bedeutung besitzt, hergestellt werden, in dem man diese gemäss Bull. Soc. Chim. Belg. 85 11 (1976) mit Natriumformiat/Ameisensäure zu einer Verbindung der Formel XIII reduziert und diese gemäss den oben beschriebenen Verfahrensweisen weiter umsetzt.

Trans-konfigurierte 2-Azabiocycloalkan-3-carbonsäuren der Formel III′, in der n die oben genannte Bedeutung hat und W′ für Wasserstoff steht, sowie ihre Alkyl- und Aralkylester können hergestellt werden indem man Enamine der Formel XVII, in welcher n vorstehende Bedeutung hat und $X^1$ für Dialkylamino mit 2 bis 10 C-Atomen oder für einen Rest der Formel XVIIa worin m und o eine ganze Zahl von 1 bis 3, $(m+o) \geqslant 3$ und A $CH_2$, NH, O oder S bedeutet, steht (Organikum, 6. Auflage, S. 370, 1967),

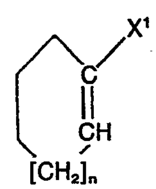

XVII

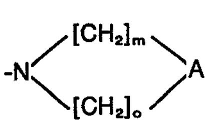

XVIIa

mit N-acylierten β-Halogen-α-amino-propionsäureestern der Formel XVIII (freie Aminoverbindung s. Helv. Chim. Acta 40, 1541 (1957)), in welcher $X^2$ für Halogen, vorzugsweise Chlor oder Brom, $Y^1$ für Alkanoyl mit 1 bis 5 C-Atomen, Aroyl mit 7 bis 9 C-Atomen oder andere, in der Peptidchemie übliche, sauer abspaltbare Schutzgruppen und $R_4'$ für Alkyl mit 1 bis 5 C-Atomen oder Aralkyl mit 7 bis 9 C-Atomen steht.

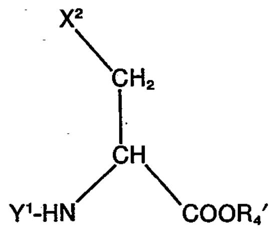

XVIII

oder mit Acrylsäureestern der Formel XIX (Chem. Ber. 91 2427 (1958)), in welcher $Y^1$ und $R_4'$ vorstehende Bedeutung haben

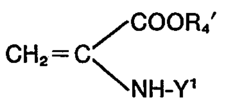

XIX

Verbindungen der Formel XX, in welcher $R_4'$ und $Y^1$ vorstehende Bedeutung haben, umsetzt,

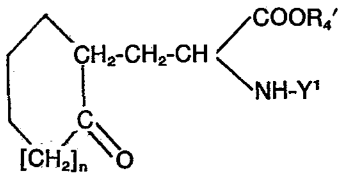

XX

diese mit Hilfe starker Säuren unter Acylamid- und Esterspaltung zu Verbindungen der Formel XXIa, die auch in der tautomeren Form der Formel XXIb vorliegen können, in welchen n vorstehende Bedeutung hat, cyclisiert,

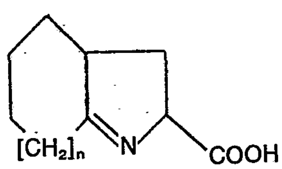

XXIa

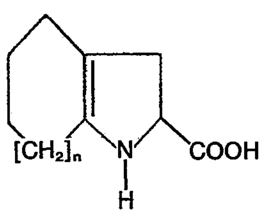

XXIb

die Verbindungen der Formel XXIa oder b, gegebenenfalls nach Überführung in ihre $C_1$–$C_{18}$-Alkyl- oder $C_7$–$C_{10}$-Aralkylester, durch katalytische Hydrierung in Gegenwart von Übergangsmetallkatalysatoren oder durch Reduktion mit Boran-Amin-Komplexen oder komplexen Borhydriden trans-konfigurierte in Verbindungen der Formel III′, in welcher n vorstehende Bedeutung hat und W′ für Wasserstoff, Alkyl mit 1 bis 18 C-Atomen oder Aralkyl mit 7 bis 10 C-Atomen steht, überführt, gegebenenfalls Ester der Formel III′ verseift, und diese, falls W′ Wasserstoff ist, gegebenenfalls zu Verbindungen der Formel III′, in welcher n vorstehende Bedeutung hat und W′ für Alkyl mit 1 bis 18 C-Atomen oder Aralkyl mit 7 bis 10 C-Atomen steht, verestert.

In J. Amer. Chem. Soc. 81 (1956) 2596 wird die Reaktion von 3-Brompropylamin mit einem Enamin des Cyclohexanons beschrieben.

Es wird weiter ausgeführt, dass für den Ablauf der Alkylierung eine freie $NH_2$-Gruppe notwendig sei. Es ist daher überaus überraschend, dass auch Halogenpropionsäure-Derivate mit acylierter Aminogruppe, wie sie die vorstehend beschriebenen Verbindungen der Formel XVIII darstellen, zur Alkylierung von Enaminen eingesetzt werden können. Dabei werden vorzugsweise Enamine des Cyclohexanons, Cycloheptanons eingesetzt. Als Aminkomponente kommt beispielsweise Diethylamin, Pyrrolidin, Piperidin oder Morpholin in Frage. Aber auch andere sekundäre Amine sind geeignet. Bevorzugt sind Pyrrolidinocycloalkylene.

Formyl, Acetyl, Propionyl oder Benzoyl oder andere sauer abspaltbare Schutzgruppen wie z. B. tert.-Butyloxycarbonyl sind besonders geeignete Gruppen $Y^1$ der β-Brom bzw. Chlor-α-aminocarbonsäureester der Formel XVIII. Vorzugsweise setzt man die ($C_1$–$C_3$)-Alkyl- oder die Benzylester ein.

Die unter den basischen Versuchsbedingungen aus den β-Halogen-α-aminopropionsäureestern intermediär entstehenden Acrylsäurederivate der Formel XIX sind ebenfalls als Ausgangsverbindung geeignet. Sie werden z. B. durch Behandeln der Halogenaminopropionsäurederivate oder der analogen O-Tosyl-Serin-Derivate mit Basen hergestellt. Vorzugsweise werden tertiäre organische Basen wie z. B. Triäthylamin verwendet. Es ist von Vorteil, unter Zusatz geringer Mengen von Polymerisationsinhibitoren wie z. B. Hydrochinon in organischen Lösemitteln zu arbeiten. Die Acrylsäurederivate der Formel XIX können anstelle der Halogenpropionsäurederivate unter identischen Reaktionsbedingungen eingesetzt werden.

Als Lösemittel für die Enaminsynthese eignen sich nicht alkylierbare organische Lösemittel, wie beispielsweise Dimethylacetamid, DMSO, THF oder Toluol. Besonders geeignet ist Dimethylformamid.

Es empfiehlt sich, die Enamine der Formel XVII im Überschuss einzusetzen und so N-Acyl-acrylester als Verunreinigung im Endprodukt zu vermeiden.

Die zur Cyclisierung notwendige Hydrolyse der N-Acylgruppe wird im allgemeinen gemeinsam mit einer Spaltung der Esterfunktion durch starke wässrige Mineralsäuren wie Schwefelsäure oder bevorzugt Salzsäure bewirkt. Im Falle der N-tert.-Butyloxycarbonyl-Derivate der Formel XVII ist es bei Verwendung von Dioxan/HCl oder wasserfreier Trifluoressigsäure beispielsweise möglich, die Esterfunktion zu erhalten, die Ester der Dehydrocarbonsäuren XXIa oder XXIb zu isolieren. Diese lassen sich durch Hydrierung in Gegenwart von Metallkatalysatoren oder Umsetzung mit Boran-Amin-Komplexen oder komplexen Borhydriden in die trans-konfigurierten 2-Azabicyclocarbonsäureester der Formel III′ überführen.

Für die katalytische Hydrierung sind Edelmetall- oder Nickelkatalysatoren geeignet. Das bei der katalytischen Hydrierung auftretende Isome-

renverhältnis ist von den Reaktionsbedingungen und der Art des verwendeten Katalysators abhängig. Eine Verkürzung der Reduktionszeit durch höheren Wasserstoffdruck ist möglich, jedoch sollte die Temperatur niedrig gehalten werden. Als Lösemittel für die katalytische Hydrierung eignen sich beispielsweise Ethanol, Methanol, Essigether, Dioxan, Eisessig oder Gemische dieser Lösemittel.

Verbindungen der oben genannten Formel III', in welcher n für eine ganze Zahl von 0 bis 3 und W' für Wasserstoff steht, sind auch durch Reduktion von Verbindungen der Formeln XXXIa oder b, in welchen n vorstehende Bedeutung hat, mit Boran-Amin-Komplexen oder komplexen Borhydriden in niederen Alkoholen zugänglich.

Als Reduktionsmittel ist Natriumborhydrid in Alkoholen, insbesondere in Methanol, Ethanol oder Isopropanol, bevorzugt. Ebenso verwendbar sind Amin-Boran-Komplexe in Eisessig.

Die Isolierung der reinen trans-Verbindungen der Formel III kann beispielsweise durch chromatographische Verfahren oder Kristallisationsverfahren erfolgen.

Die reine trans-Verbindung der Formel III' wird vorteilhafterweise durch fraktionierte Kristallisation aus dem Diastereomerengemisch der Amin-Boran-Komplex- oder Boranat-Reduktion abgetrennt.

Die Verbindungen der Formeln III' können gegebenenfalls nach Methoden, wie sie z.B. in Houben-Weyl, Bd. VIII (1952) beschrieben sind, in die $(C_1–C_{18})$-Alkyl- oder $(C_7–C_{10})$-Aralkylester überführt werden.

Die als Ausgangsstoffe zur Herstellung der Verbindungen der Formel I verwendeten Verbindungen der Formel II mit Y, Z = Wasserstoff, $R_1$ = Methyl und $R_2$ = Methyl oder Äthyl und X = Phenyl sind bekannt (EP-A-0 037 231).

Die Verbindungen der Formel II lassen sich nach verschiedenen Verfahrensweisen herstellen. Eine Synthesevariante geht von einem Keton der obengenannten Formel VII aus, das nach bekannten Verfahrensweisen in einer Mannich-Reaktion mit einer Verbindung der obengenannten Formel VI zusammen mit Aminosäureestern der Formel XXII,

$$H_2N\text{-}\underset{|\atop R_1}{CH}\text{-}CO_2W \qquad XXII$$

$$WO_2C\text{-}\underset{|\atop R_1}{\overset{*}{C}H}\text{-}NH\text{-}\underset{|\atop CO_2R_2}{\overset{*}{C}H}\text{-}CH_2\text{-}CO\text{-}X \qquad XXIII$$

worin $R_1$ und W die obengenannten Bedeutungen besitzen, zu einer Verbindung der Formel XXIII, worin $R_1$, $R_2$, X und W die obengenannten Bedeutungen besitzen, mit der Einschränkung, dass im Falle W = ein hydrogenolytisch abspaltbarer Rest, insbesondere Benzyl bedeutet, $R_2$ nicht die Bedeutung von W besitzen darf, umsetzt. Spaltet man den Rest W hydrogenolytisch mit Hilfe von beispielsweise Palladium ab, werden Verbindungen der Formel II mit Y, Z jeweils = Wasserstoff enthalten. Wird der Rest W mit Säuren wie beispielsweise Trifluoressigsäure oder Salzsäure in einem inerten organischen Lösungsmittel wie beispielsweise Dioxan abgespalten, erhält man Verbindungen der Formel II mit Y und Z zusammen = Sauerstoff.

Verbindungen der Formel XXIII sind auch durch Michael-Addition einer Verbindung der obengenannten Formel V mit einer Verbindung der obengenannten Formel XXII nach bekannten Verfahrensweisen zugänglich. Bevorzugt eignet sich dieses Verfahren zur Herstellung von solchen Verbindungen der Formel XXIII, in denen $R_1$ = Methyl, $R_2$ = Äthyl und X = Aryl bedeuten.

Die Verbindungen der Formel XXIII fallen als Diastereomerengemische an. Bevorzugte Diastereomeren der Formel XXIII sind solche, in denen die mit einem Stern markierten chiralen C-Atome jeweils S-Konfiguration aufweisen. Diese können durch Umkristallisieren oder durch Chromatographie, beispielsweise an Kieselgel, abgetrennt werden. Bei der nachfolgenden Abspaltung des Restes W bleiben die Konfigurationen der chiralen C-Atome erhalten.

Die als Ausgangsstoffe zur Herstellung der Verbindungen der Formel I verwendeten Verbindungen der obengenannten Formel IV werden aus den Verbindungen der obengenannten Formel III durch Umsetzen mit einer N-geschützten 2-Aminocarbonsäure der Formel XXIV

$$V\text{-}HN\text{-}\underset{|\atop R_1}{CH}\text{-}CO_2H \qquad XXIV$$

worin V eine Schutzgruppe ist und $R_1$ die obengenannte Bedeutung besitzt, nach bekannten Verfahrensweisen erhalten. Als Schutzgruppe V, die nach beendeter Reaktion wieder abgespalten wird, kommen beispielsweise die Gruppen Benzyloxycarbonyl oder tert.-Butoxycarbonyl in Frage.

Die Umsetzung einer Verbindung der Formel II mit einer Verbindung der Formel III zur Herstellung einer Verbindung der Formel I erfolgt gemäss einer in der Peptidchemie bekannten Kondensationsreaktion, wobei als Kondensationsmittel beispielsweise Dicyclohexylcarbodiimid und 1-Hydroxybenzotriazol zugesetzt werden. Bei der nachfolgenden hydrogenolytischen Abspaltung des Restes W wird als Katalysator bevorzugt Palladium verwendet, während bei der sauren Abspaltung des Restes W als Säuren bevorzugt Trifluoressigsäure oder Chlorwasserstoff eingesetzt werden.

In den oben beschriebenen Reaktionen zur Herstellung der Verbindungen der Formeln III', IV und I bleiben jeweils die Konfigurationen der Zwischenprodukte an den Brückenkopf C-Atomen 3a und (6+n)a erhalten. Im Falle der trans-Konfiguration der H-Atome an den C-Atomen 3a und 6+n)a werden die betreffenden Verbindungen teils als reine Diastereomere, teils als Gemische

von Diastereomeren erhalten, die wie oben angegeben, die 2β, 3aα, (6+n)aβ bzw. die 2β, 3aβ, (6+n)aα-Konfiguration aufweisen. Diese Isomeren können leicht durch Umkristallisation oder Chromatographie getrennt werden.

Die gemäss oben beschriebenen Verfahrensweisen erhaltenen Verbindungen der Formel III' fallen als racemische Gemische an und können als solche in die weiteren oben beschriebenen Synthesen eingesetzt werden. Sie können aber auch nach Auftrennung der Racemate mit üblichen Methoden beispielsweise über Salzbildung mit optisch aktiven Basen oder Säuren in die optischen Antipoden als reine Enantiomere eingesetzt werden.

Fallen die Verbindungen der Formel I als Racemate an, können auch diese nach den üblichen Methoden wie beispielsweise über Salzbildung mit optisch aktiven Basen oder Säuren in ihre Enantiomeren gespalten werden.

Die erfindungsgemässen Verbindungen der Formel I liegen als innere Salze vor. Als amphotere Verbindungen können sie Salze mit Säuren oder Basen bilden. Diese Salze werden in üblicher Weise durch Umsetzen mit einem Äquivalent Säure bzw. Base hergestellt.

Die Verbindungen der Formel I und deren Salze besitzen lang andauernde, intensive blutdrucksenkende Wirkung. Sie sind starke Hemmer des Angiotensin-Converting-Enzyms (ACE-Hemmer). Sie können zur Bekämpfung des Bluthochdrucks verschiedener Genese eingesetzt werden. Auch ihre Kombination mit anderen blutdrucksenkenden, gefässerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1–100 mg, vorzugsweise bei 1–40 mg je Einzeldosis bei einem normalgewichtigen erwachsenen Patienten. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemässen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wässrige, alkoholische oder ölige Suspensionen oder wässrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Äthanol, Propandiol oder Glycerin, daneben auch Zuckerlösungen wie Glukose- oder Mannitlösungen, oder auch eine Mischung aus den verschiedenen genannten Lösungsmitteln.

Die ausserordentlich starke Wirksamkeit der Verbindungen gemäss Formel I wird durch die pharmakologischen Daten der nachfolgenden Tabelle belegt:

Intraduodenale Gabe an der narkotisierten Ratte, 50% Hemmung der durch 310 ng Angiotensin I ausgelösten Presserreaktion 30 min. nach Applikation in der Dosis ..... = $ED_{50}$:

Tabelle

H-Atome an C–3a und C–(6+n)a besitzen trans-Konfiguration; X = Phenyl, $R_1$ = Methyl

| n | Y | Z | $R_2$ | Konfiguration des Bicyclus | $ED_{50}$ (µg/kg) |
|---|---|---|---|---|---|
| 0 | H | H | H | 2S,3aR,6aS | 700 |
| 0 | H | H | $C_2H_5$ | 2S,3aR,6aS | 40 |
| 1 | H | H | H | 2S,3aR,7aS | 800 |
| 1 | H | H | $C_2H_5$ | 2S,3aR,7aS | 55 |
| 1 | H | H | H | 2S,3aS,7aR | 850 |
| 1 | H | H | $C_2H_5$ | 2S,3aS,7aR | 65 |
| 2 | H | H | H | 2S,3aR,8aS | 1080 |
| 2 | H | H | $C_2H_5$ | 2S,3aR,8aS | 110 |
| 1 | –O– | | $C_2H_5$ | 2S,3aR,7aS | 180 |

Die Symbole n, X, Y, Z, $R_1$ und $R_2$ beziehen sich auf die Verbindungen der Formel I.

Die nachfolgenden Beispiele dienen zur Erläuterung der Erfindung, ohne diese auf die stellvertretend genannten Verbindungen zu beschränken:

Beispiel 1 (Referenzbeispiel)
N-(1-S-Carbethoxy-3-oxo-3-phenyl-propyl)-S-alanyl-2β,3aβ,8aβ-decahydrocyclohepta[b]-pyrrol-2-carbonsäure-benzylester

10 mMol Acetophenon, 10 mMol Glyoxylsäureäthylester und 10 mMol S-Alanyl-2S,3aR,8aR-decahydrocyclohepta-[b]pyrrol-2-carbonsäurebenzylester werden in 30 ml Eisessig 36 Stunden auf 45 °C erhitzt. Nach Einengen im Vakuum wird mit wässrigem Natriumbicarbonat alkalisch gestellt und mit Essigester extrahiert. Die Essigesterphase wird eingeengt und an Kieselgel chromatographiert.

Beispiel 2
N-(1-S-Carbethoxy-3-phenyl-propyl)-S-alanyl-2S,3aR,7aS-octahydroindol-2-carbonsäure

a) DL-2β,3aβ,7aα-Octahydroindol-2-carbonsäure-benzylester-Hydrochlorid

Zu einer bei −5 bis 0 °C hergestellten Lösung von 1,4 ml Thionylchlorid in 14 ml Benzylalkohol werden bei −10° bis 0 °C 1,4 g DL-2β,2β,3aβ,7aα-Octahydroindol-2-carbonsäure gegeben. Man rührt 1 Stunde bei 0 °C und lässt über Nacht bei 20 bis 25 °C stehen. Der Benzylalkohol wird im Hochvakuum bei 50 °C abdestilliert und der Rückstand mit Diisopropyläther digeriert. Man erhält 2,5 g farblose Kristalle vom Fp. 154 °C.

b) N-(1-S-Carbethoxy-3-phenyl-propyl)-S-alanyl-2β,3aβ,7aα-octahydroindol-2-carbonsäure-benzylester

Zu einer Suspension von 2,16 g N-(1-S-Carbethoxy-3-phenyl-propyl)-S-alanin in 8,6 ml wasserfreiem Dimethylformamid werden 1,06 g 1-Hydroxy-benzotriazol, 2,2 g
DL-2β,3aβ,7aα-Octahydroindol-2-carbonsäure-benzylester-Hydrochlorid
sowie 1,08 ml N-Äthylmorpholin und bei 0 °C 1,7 g Dicyclohexylcarbodiimid gegeben. Nach 3,5 stündigem Rühren bei 20 bis 25 °C wird das Reaktionsgemisch mit 20 ml Essigester verdünnt und der abgeschiedene Dicyclohexylharnstoff abfiltriert. Nach Einengen im Vakuum wird der Rückstand in Ether aufgenommen, zweimal mit gesättigtem wässrigem Natriumbicarbonat gewaschen, über Natriumsulfat getrocknet und eingedampft. Nach Chromatographie an Kieselgel mit Essigester-Cyclohexan als Laufmittel erhält man 2 blassgelbe Öle im Verhältnis 1:1, die jeweils ein Isomeres der gewünschten Verbindung enthalten.

$^1$H-NMR-Daten des Isomeren mit 2R,3aR,7aS-Konfiguration:

7,35 (s, 5H); 7,2 (s, 5H); 5,18 (s, 2H); 4,55 (d, 1H); 4,1 (q, 2H); 3,4–2,3 (m, 6H); 2,4–1,3 (m, 12H); 1,3 (t, 3H); 1,1 (d, 3H).

$^1$H-NMR-Daten des Isomeren mit 2S,3aS,7aR-Konfiguration:

7,35 (s, 5H); 7,2 (s, 5H); 5,16 (s, 2H); 4,9–4,2 (m, 1H); 4,2 (q, 2H); 3,9–2,4 (m, 6H); 2,4–1,4 (m, 12H); 1,25 (d+t, 6H).

c) N-(1-S-Carbethoxy-3-phenyl-propyl)-S-alanyl-2S,3aR,7aS-octahydroindol-2-carbonsäure

1,7 g des unter b) erhaltenen Isomeren mit 2S, 3aS,7aR-Konfiguration werden in 60 ml wasserfreiem Ethanol unter Zusatz von 200 mg Palladium-Kohle (10%) bei 20 bis 25 °C unter Normaldruck während 2 Stunden hydriert. Der Katalysator wird abfiltriert und das Filtrat eingeengt. Man erhält 1,2 g der Titelverbindung als farblosen Schaum.

$^1$H-NMR-Daten:

7,2 (s, 5H); 4,4 (m, 4H); 4,2 (q, 2H); 3,6–1,3 (m, 18H); 1,28 (d+t, 6H).

Das Hydrochlorid der obengenannten Verbindung wird als farbloses amorphes Pulver erhalten.

Beispiele 3 bis 7

Die in diesen Beispielen genannten Verbindungen werden analog den in Beispiel 2 beschriebenen Verfahrensweisen hergestellt.

Beispiel 3

N-(1-S-Carbethoxy-3-phenyl-propyl)-S-alanyl-2S, 3aS,7aR-octahydroindol-2-carbonsäure

Diese Verbindung erhält man aus dem Produktgemisch von Beispiel 11, das man nach der Verfahrensweise von Beispiel 2 weiter umsetzt. Vor der Hydrierung gemäss Beispiel 2c werden die Isomeren durch Säulenchromatographie an Kieselgel getrennt.

$^1$H-NMR-Daten:

7,2 (s, 5H); 4,0–4,6 (m, 4H); 4,15 (q, 2H); 3,8–1,2 (m, 18H); 1,3 (t, 3H); 1,2 (d, 3H).

Das Hydrochlorid wird als farbloses, amorphes Pulver erhalten.

Beispiel 4

N-(1-S-Carbethoxy-3-phenyl-propyl)-S-alanyl-2S, 3aR,6aS-octahydrocyclopenta[b]pyrrol-2-carbonsäure

$^1$H-NMR-Daten:

7,25 (s, 5H); 4,35 (m, 4H); 4,2 (q, 2H); 3,9–1,4 (m, 16H); 1,35 (t, 3H); 1,15 (d, 3H).

Das Hydrochlorid wird als farbloses, amorphes Pulver erhalten.

Beispiel 5

N-(1-S-Carbethoxy-3-phenyl-propyl)-S-alanyl-2S, 3aS,6aR-octahydrocyclopenta[b]pyrrol-2-carbonsäure

$^1$H-NMR-Daten:

7,3 (s, 5H); 4,3 (m, 4H); 4,2 (q, 2H); 3,8–1,3 (m, 16H); 1,3 (t+d, 6H).

Das Hydrochlorid wird als farbloses, amorphes Pulver erhalten.

Beispiel 6

N-(1-S-Carbethoxy-3-phenyl-propyl)-S-alanyl-2S,3aR,8aS-decahydrocyclohepta[b]pyrrol-2-carbonsäure

$^1$H-NMR-Daten:

7,15 (s, 5H); 4,4 (m, 4H); 4,2 (q, 2H); 3,8–1,3 (m, 20H); 1,3 (t+d, 6H).

Beispiel 7

N-(1-S-Carbethoxy-3-phenyl-propyl)-S-alanyl-2S,3aS,8aR-decahydrocyclohepta[b]pyrrol-2-carbonsäure

$^1$H-NMR-Daten:

7,2 (s, 5H); 4,4 (m, 4H); 4,25 (q, 2H); 3,8–1,2 (m, 20H); 1,3 (t, 3H); 1,15 (d, 3H).

Beispiel 8

2β,3aβ,7aα-Octahydroindol-carbonsäure

a) 3-Chlor-N-acetyl-alanin-methylester

181 g 3-Chlor-alanin-methylester-Hydrochlorid werden mit 163,9 g Acetylchlorid in 1,5 l wasserfreiem Toluol ca. 5 Stunden unter Rückfluss erhitzt, bis eine klare Lösung entstanden ist. Man engt zur Trockene ein und kristallisiert den Rückstand aus Essigester/Petroläther. Es werden 170 g Produkt vom Fp. 104 °C erhalten

b) 3-(2-Oxo-cyclohexyl)-N-acetyl-alanin-methylester

Man löst 160 g 3-Chlor-N-acetyl-alanin-methylester und 171,9 g 1-Pyrrolidino-cyclohexen in 1,2 l

absolutem DMF und lässt das Gemisch 3 Tage bei 20 bis 25 °C stehen.

Die Lösung wird im Hochvakuum eingeengt, der erhaltene Rückstand in 600 ml Wasser aufgenommen und mit konz. Salzsäure auf einen pH-Wert von 2,0 gestellt. Man extrahiert die wässrige Lösung mittels Essigester, trocknet die organische Phase über Natriumsulfat und engt ein. Das Produkt wird als gelbes Öl erhalten.

c) 3,3a,4,5,6,7-2H-Hexahydroindol-2-carbonsäure-Hydrochlorid

220 g der unter b) erhaltenen Verbindung wird mit 1 l 2n Salzsäure 2 Stunden am Rückfluss zum Sieden erhitzt. Man extrahiert das Reaktionsgemisch mit Essigester und engt die wässrige Phase ein. Restmengen an Wasser werden durch dreimaliges Eindampfen im Vakuum unter Zusatz von Toluol entfernt. Es werden 210 g des Produktes als gelbes Öl erhalten, das beim Stehenlassen kristallisiert.

d) 2β,3aβ,7aα-Octahydroindol-2-carbonsäure

128 g 3,3a,4,5,6,7-2H-Hexahydroindol-2-carbonsäure-Hydrochlorid werden in 700 ml Eisessig unter Zusatz von 4 g Platin/Kohle (10%) unter Normaldruck bei Raumtemperatur hydriert.

Man filtriert den Katalysator ab und engt das Filtrat zur Trockene ein. Der Rückstand wird in 500 ml heissem Ethanol gelöst und auf −20 °C gekühlt. Hierbei fällt das 2β,3aβ,7aβ-Isomere der Titelverbindung aus. Aus der Lösung erhält man das Produkt durch Einengen und Versetzen mit Isopropanol in Form farbloser Kristalle vom Fp. 280 °C.

Beispiel 9

2β,3aβ,8aα-Decahydro-cyclohepta[b]pyrrol-2-carbonsäure

Diese Verbindung wird ausgehend von Pyrrolidino-cyclohepten analog den Verfahrensweisen b) bis d) von Beispiel 8 hergestellt.

Beispiel 10

2β,3aβ,6aα-Octahydro-cyclopenta[b]pyrrol-2-carbonsäure

Diese Verbindung wird ausgehend von Pyrrolidino-cyclopenten analog den Verfahrensweisen b) bis d) von Beispiel 8 hergestellt.

Beispiel 11

2β,3aα,7aβ-Octahydroindol-2-carbonsäure

a) 3,4,5,6,7,8-Octahydro-1H-chinolin-2on

392 g Cyclohexanon und 212 g Acrylnitril werden zusammen mit 20 g Cyclohexylamin und 4 g Eisessig sowie 0,4 g Hydrochinon 4 Stunden bis zu einer Endtemperatur von 200 °C unter Rückfluss erhitzt. Das nach Destillation bei 100 bis 150 °C/0,5 mm Hg erhaltene Destillat wird 2 Tage mit 10 ml 50%iger Essigsäure auf 200 °C erhitzt. Das Reaktionsgemisch wird nach Abkühlen aus Methanol/Wasser umkristallisiert. Zusammen mit dem oben erhaltenen Destillationsrückstand der aus n-Hexan umkristallisiert wird, werden 460 g des Produktes vom Fp. 143 bis 144 °C erhalten.

b) trans-Octahydro-1H-chinolin-2on

Ein Gemisch aus 25 g des unter a) erhaltenen Produktes und 70 g Natriumformiat in 120 ml Ameisensäure wird 18 Stunden am Rückfluss zum Sieden erhitzt. Die Reaktionslösung wird mit 20%igem wässrigem Natriumhydroxid alkalisch gestellt und mit Essigester extrahiert. Die organische Phase wird über Natriumsulfat getrocknet und eingedampft. Der Rückstand wird aus Cyclohexan umkristallisiert, wobei das Produkt vom Fp. 152 °C erhalten wird.

c) 3,3-Dichlor-trans-octahydro-1H-chinolin-2on

Zu einer Lösung von 19,4 g der unter b) erhaltenen Verbindung und 24,3 g Phosphorpentachlorid in 300 ml wasserfreiem Chloroform wird eine Lösung von 36,4 g Sulfurylchlorid in 40 ml Chloroform bei 20 bis 30 °C während 30 Minuten zugetropft. Das Gemisch wird 6 Stunden zum Sieden erhitzt und über Nacht bei 20 bis 25 °C stehen gelassen.

Man neutralisiert mit eiskaltem gesättigtem wässrigem Kaliumcarbonat, extrahiert das Gemisch mit Methylenchlorid, trocknet die organische Phase über Natriumsulfat und engt diese ein. Nach Umkristallisation des Rückstandes aus Ethanol unter Zusatz von Aktivkohle werden 25 g des Produktes von Fp. 195 bis 198 °C erhalten.

d) 3-Chlor-trans-octahydro-1H-chinolin-2on

15,6 g der unter c) erhaltenen Verbindung werden in 1 l Ethanol und 9,7 ml Triäthylamin unter Zusatz von Raney-Nickel bei 20 bis 25 °C unter Normaldruck bis zur Aufnahme von 1 Mol-Äquivalent Wasserstoff hydriert.

Nach Abfiltrieren des Katalysators wird das Filtrat zur Trockene eingeengt und der Rückstand in Essigester aufgenommen. Die organische Phase wird zweimal mit Wasser gewaschen, über Natriumsulfat getrocknet und eingeengt. Der Rückstand wird mit Diisopropyläther verrieben, abgesaugt und getrocknet. Man erhält das Produkt in Form blassgelber Kristalle von Fp. 115–120 °C.

e) 2β,3aα,7aβ-Octahydroindol-2-carbonsäure

3,75 g der unter d) erhaltenen Verbindung werden zu einer siedenden Lösung von 6,63 g Bariumhydroxid-Octahydrat in 120 ml Wasser gegeben. Nach 4stündigem Erhitzen unter Rückfluss werden 0,9 ml konz. Schwefelsäure zugesetzt, eine zusätzliche Stunde unter Rückfluss erhitzt, die Reaktionslösung abfiltriert und das Filtrat mittels 1n Natriumhydroxid auf einen pH-Wert von 6,5 gestellt. Nach Eindampfen der Lösung wird der Rückstand in Ethanol erhitzt, erneut abfiltriert und das Filtrat auf ein kleines Volumen eingeengt. Beim Abkühlen wird ein kristallines 1:1-Gemisch der Titelverbindung mit der Verbindung des Bsp. 48 vom Fp. 275–276 °C erhalten.

Beispiel 12

2β,3aβ,6aα-Octahydrocyclopenta[b]pyrrol-2-carbonsäure

a) 1,2,3,4,6,7-Hexahydro-5H-1-pyrind-2-on

Ein Gemisch aus 1 Mol Cyclopentanon, einem

Mol Acrylnitril, 0,05 Mol Ammoniumacetat und 3 ml 30%igem Ammoniak wird in einem Druckgefäss 3 Stunden auf 220 °C erhitzt. Man filtriert das Gemisch über Kieselgel mit Essigester/Cyclohexan (1:1) und kristallisiert den nach Eindampfen des Filtrats erhaltenen Rückstand aus Cyclohexan um. Das Produkt schmilzt bei 118 bis 120 °C.

$^1$H-NMR-Daten:

9,4 (s-breit, 1H); 3,2–2,0 (m, 12H).

b) Octahydro-trans-5H-1-pyrind-2-on

Diese Verbindung wird analog der in Beispiel 11 unter b) beschriebenen Verfahrensweise hergestellt.

$^1$H-NMR-Daten:

7,8 (s-breit, 1H); 2,9 (s-breit, 1H); 2,6–2,2 (m, 2H); 2,1–1,0 (m, 8H).

c) 3,3-Dichlor-octahydro-trans-5H-1-pyrind-2-on

Diese Verbindung wird analog der in Beispiel 11 unter c) beschriebenen Verfahrensweise hergestellt.

$^1$H-NMR-Daten:

7,9 (s-breit, 1H); 3,8 (s-breit, 1H); 3,2–2,0 (m, 2H); 2,1–1,0 (m, 6H).

d) 3-Chlor-octahydro-trans-5H-1-pyrind-2-on

Diese Verbindung wird analog der in Beispiel 11 unter d) beschriebenen Verfahrensweise hergestellt.

$^1$H-NMR-Daten:

7,8 (s-breit, 1H); 4,6–4,3 (m, 1H); 3,3–3,0 (m, 1H); 2,1 (d, J = 6 Hz, 2H); 1,8–1,1 (m, 6H).

e) 2β,3aβ,6aα-Octahydrocyclopenta[b]pyrrol-2-carbonsäure

Diese Verbindung wird analog der in Beispiel 11 unter e) beschriebenen Verfahrensweise hergestellt.

$^1$H-NMR-Daten:

4,7–4,4 (m, 1H); 3,0–0,9 (m, 10H).

Beispiel 13

2β,3aβ,8aα-Decahydrocyclohepta[b]pyrrol-2-carbonsäure

a) 1,2,3,4,5,6,7,8-Octahydrocyclohepta[b]-pyrid-2-on

Diese Verbindung wird analog der in Beispiel 11 unter a) beschriebenen Verfahrensweise ausgehend von Cycloheptanon hergestellt.

$^1$H-NMR-Daten:

9,4 (s-breit, 1H); 3,2–2,0 (m, 14H).

b) trans-Decahydrocyclohepta[b]pyrid-2-on

Diese Verbindung wird analog der in Beispiel 11 unter b) beschriebenen Verfahrensweise ausgehend von der unter a) vorgenannten Verbindung hergestellt.

$^1$H-NMR-Daten:

7,9 (s-breit, 1H); 2,9 (s-breit, 1H); 2,6–2,2 (m, 2H); 2,1–1,0 (m, 12H).

c) 3,3-Dichlor-trans-decahydrocyclohepta[b]-pyrid-2-on

Diese Verbindung wird analog der in Beispiel 11 unter c) beschriebenen Verfahrensweise hergestellt.

$^1$H-NMR-Daten:

7,9 (s-breit, 1H); 3,8 (s-breit, 1H); 3,2–2,0 (m, 2H); 2,1–1,0 (m, 10H).

d) 3-Chlor-trans-decahydro-cyclohepta[b]-pyrid-2-on

Diese Verbindung wurde analog der in Beispiel 11 unter d) beschriebenen Verfahrensweise hergestellt.

$^1$H-NMR-Daten:

7,8 (s-breit, 1H); 4,6–4,3 (m, 1H); 3,3–3,0 (m, 1H); 2,1 (d, J = 6Hz, 2H); 1,8–1,1 (m, 10H).

e) 2β,3aβ,8aα-Decahydrocyclohepta[b]pyrrol-2-carbonsäure

Diese Verbindung wurde analog der in Beispiel 11 unter e) beschriebenen Verfahrensweise hergestellt.

$^1$H-NMR-Daten:

4,7–4,4 (m, 1H); 3,2–1,1 (m, 1H).

Beispiel 14

a) 2β,3aα,7aβ-Octahydroindol-2-carbonsäure-tert.butylester-Hydrochlorid

Diese Verbindung wurde aus 2β,3aα,7aβ-Octahydroindol-2-carbonsäure analog der in Beispiel 14b) beschriebenen Verfahrensweise hergestellt.

$^1$H-NMR-Daten:

4,7–4,4 (m, 1H); 3,2–1,1 (m, 12H); 1,2 (s, 9H).

Die Verbindungen der folgenden Beispiele 15 bis 19 wurden analog der in Beispiel 14 beschriebenen Verfahrensweise hergestellt.

b) 2β,3aβ,7aβ-Octahydroindol-2-carbonsäure-tert.butylester-Hydrochlorid

Zu einer auf −10 °C gekühlten Lösung von 10 g 2β,3aβ,7aβ-Octahydroindol-2-carbonsäure in 100 ml Dioxan werden 10 ml konz. Schwefelsäure und 50 g Isobutylen gegeben. Das Reaktionsgemisch wird im Autoklaven langsam auf 20 bis 25 °C erwärmt und 20 Stunden bei dieser Temperatur gerührt.

Man gibt das Gemisch in eiskaltes 50%iges wässriges Natriumhydroxid und extrahiert mit Methylenchlorid. Die vereinigten organischen Phasen werden mit Wasser gewaschen, mit Natriumsulfat getrocknet und eingeengt. Der Rückstand wird in Ether aufgenommen und mittels etherischem Chlorwasserstoff auf einen pH-Wert von 2,0 bis 3,0 gestellt. Man engt zur Trockene ein und verreibt das Produkt mit Diisopropylether. Nach Absaugen werden 7,3 g der Titelverbindung erhalten.

$^1$H-NMR-Daten:

1,0–2,5 (m, 11H); 1,3 (s, 9H); 3,4–3,9 (m, 1H); 4,0–4,5 (m, 1H).

Beispiel 15

2β,3aβ,7aα-Octahydroindol-2-carbonsäure-tert.butylester-Hydrochlorid

$^1$H-NMR-Daten:

4,7–4,3 (m, 1H); 3,4–1,1 (m, 12H); 1,2 (s, 9H).

Beispiel 16

2β,3aα,6aβ-Octahydrocyclopenta[b]pyrrol-2-carbonsäure-tert.butylester-Hydrochlorid

$^1$H-NMR-Daten:

4,8–4,4 (m, 1H); 3,4–1,2 (m, 10H); 1,2 (s, 9H).

Beispiel 17

2β,3aβ,6aα-Octahydrocyclopenta[b]pyrrol-2-carbonsäure-tert.butylester-Hydrochlorid

$^1$H-NMR-Daten:

4,8–4,4 (m, 1H); 3,5–1,2 (m, 10H); 1,2 (s, 9H).

Beispiel 18

2β,3aα,8aβ-Decahydrocyclohepta[b]pyrrol-2-carbonsäure-tert.butylester-Hydrochlorid

$^1$H-NMR-Daten:

4,7–4,4 (m, 1H); 3,3–1,0 (m, 14H); 1,2 (s, 9H).

Beispiel 19

2β,3aβ,8aα-Decahydrocyclohepta[b]pyrrol-2-carbonsäure-tert.butylester-Hydrochlorid

$^1$H-NMR-Daten:

4,7–4,3 (m, 1H); 3,2–0,8 (m, 14H); 1,2 (s, 9H).

Beispiel 20

N-(S-Alanyl)-2S,3aR,7aS-octahydroindol-2-carbonsäure-tert.butylester-Hydrochlorid

a) N-(N'-Benzyloxycarbonyl-S-alanyl)-2S-3aR,7aS-octahydroindol-2-carbonsäure-tert.-butylester

Diese Verbindung wurde mittels Z-Alanin analog der in Beispiel 20c) beschriebenen Verfahrensweise hergestellt und wie dort beschrieben von seinen Isomeren getrennt.

$^1$H-NMR-Daten:

7,4 (s, 5H); 5,3–4,8 (m, 1H); 4,2–3,7 (m, 2H); 3,1–1,4 (m, 11H); 1,3 (d, J = 7 Hz, 3H); 1,2 (s, 9H).

b) Die vorgenannte unter a) erhaltene Verbindung wird mit Palladium/Bariumsulfat in 1n ethanolischem Chlorwasserstoff hydriert, wobei die gewünschte Titelverbindung erhalten wird.

$^1$H-NMR-Daten:

5,2–4,8 (m 1H); 4,2–3,7 (m, 2H); 3,1–1,4 (m, 11H); 1,3 (d, J = 7 Hz, 3H); 1,2 (s, 9H).

Die Verbindungen der nachfolgenden Beispiele 21 bis 25 werden analog den unter a) und b) in Beispiel 20 beschriebenen Verfahrensweisen hergestellt.

c) N-tert.Butoxycarbonyl-S-alanyl-2S,3aR,7aR-octahydroindol-2-carbonsäure-benzylester

Zu einer Lösung von 19 g Boc-Ala-OH in 100 ml DMF werden 13 ml N-Ethylmorpholin, 13,5 g 1-Hydroxy-benzotriazol und 29,6 g 2β,3aβ,7aβ-octahydroindol-2-carbonsäure-benzylester-Hydrochlorid gegeben. Das Gemisch wird im Eisbad gekühlt und mit 21 g Dicyclohexylcarbodiimid versetzt. Man rührt 15 Stunden bei 20 bis 25°C. Der ausgefallene Harnstoff wird abgesaugt, das Filtrat im Vakuum eingeengt und in Essigester aufgenommen. Die organische Phase wird je 3 mal mit wässrigem Kaliumhydrogensulfat, Kaliumhydrogencarbonat und Natriumchlorid extrahiert, getrocknet und eingedampft. Der Rückstand wird an Kieselgel mit Essigester/Cyclohexan (1:3) chromatographiert. Die erste Fraktion enthält das gewünschte Produkt. Ausbeute: 21 g

$^1$H-NMR-Daten:

1,3 (d, 3H); 1,45 (s, 9H); 1,1–2,4 (m, 12H); 3,2–3,9 (m, 2H); 5,3 (s, 2H); 7,4 (s, 5H).

Beispiel 21

N-(S-Alanyl)-2S,3aS,7aR-octahydroindol-2-carbonsäure-tert.butylester-Hydrochlorid

$^1$H-NMR-Daten:

5,2–4,7 (m, 1H); 4,4–3,8 (m, 2H); 3,1–1,4 (m, 11H); 1,25 (d, J = 7 Hz, 3H); 1,2 (s, 9H).

Beispiel 22

N-(S-Alanyl)-2S,3aR,6aS-octahydrocyclopenta-[b]pyrrol-2-carbonsäure-tert.butylester-Hydrochlorid

$^1$H-NMR-Daten:

5,3–4,7 (m, 1H); 4,4–3,8 (m, 2H); 3,1–1,4 (m, 9H); 1,3 (d, J = 7 Hz, 3H); 1,2 (s, 9H).

Beispiel 23

N-(S-Alanyl)-2S,3aS,6aR-octahydrocyclopenta-[b]pyrrol-2-carbonsäure-tert.butylester-Hydrochlorid

$^1$H-NMR-Daten:

5,3–4,7 (m, 1H); 4,4–3,8 (m, 2H); 3,1–1,4 (m, 9H); 1,3 (d, J = 7 Hz, 3H); 1,2 (s, 9H).

Beispiel 24

N-(S-Alanyl)-2S,3aR,8aS-decahydrocyclohepta-[b]pyrrol-2-carbonsäure-tert.butylester-Hydrochlorid

$^1$H-NMR-Spektrum:

5,1–4,6 (m, 1H); 4,5–3,7 (m, 2H); 3,1–1,4 (m, 13H); 1,3 (d, J = 7 Hz, 3H); 1,2 (s, 9H).

Beispiel 25

N-(S-Alanyl)-2S,3aS,8aR-decahydrocyclohepta-[b]pyrrol-2-carbonsäure-tert.butylester-Hydrochlorid

$^1$H-NMR-Daten:

5,1–4,6 (m, 1H); 4,5–3,7 (m, 2H); 3,1–1,4 (m, 13H); 1,3 (d, J = 7 Hz, 3H); 1,2 (s, 9H).

Beispiel 26

a) N-(1S-Carbethoxy-3-phenyl-propyl)-S-alanyl-2S,3aR,7aS-octahydroindol-2-carbonsäure

Diese Verbindung wird analog der in Beispiel 26 b) beschriebenen Verfahrensweise ausgehend von N-(S-Alanyl)-2S,3aR,7aS-octahydroindol-2-carbonsäure-tert.butylester hergestellt. Die Abspaltung der tert.Butylgruppe erfolgt mit Trifluoressigsäure.

b) N-(1-S-Carbethoxy-3-phenyl-propyl)-S-alanyl-2S,3aR,7aR-octahydroindol-2-carbonsäure-benzylester

10 mMol S-Alanyl-2S,3aR,7aR-octahydroindol-2-carbonsäure-benzylester werden in 30 ml wasserfreiem Ethanol gelöst. Man stellt die Lösung mittels ethanolischem Kaliumhydroxid auf einen pH-Wert von 7,0 und gibt 1 g pulverisiertes Molekularsieb (4Å) und anschliessend 10 mMol 2-Keto-4-phenyl-buttersäure-äthylester hinzu. Es wird eine Lösung von 1 g Natriumcyanborhydrid in 10 ml wasserfreiem Ethanol langsam zugetropft. Nach einer Reaktionszeit von 20 Stunden bei 20 bis 25 °C wird die Reaktionslösung filtriert und das Lösungsmittel abdestilliert. Der Rückstand wird in Essigester/Wasser aufgenommen. Nach Eindampfen der Essigesterphase wird der Rückstand an Kieselgel mit Essigester/Cyclohexan (1:4) chromatographiert.

Beispiel 27

a) N-(1S-Carbethoxy-3-keto-3-phenyl-propyl)-S-alanyl-2S,3aR,7aS-octahydroindol-2-carbonsäure

Diese Verbindung wird analog der in Beispiel 27 b) beschriebenen Verfahrensweise ausgehend von N-(S-Alanyl)-2S,3aR,7aS-octahydroindol-2-carbonsäure-tert.butylester hergestellt. Die Abspaltung der tert.Butylgruppe erfolgt mit Trifluoressigsäure.

$^1$H-NMR-Daten:

1,2 (d+t, 6H); 1,3–3,6 (m, 16H); 4,2 (q, 2H); 4,1–4,6 (m, 4H); 7,3–8,1 (m, 5H).

b) N-(1-S-Carbethoxy-3-oxo-3-phenyl-propyl)-S-alanyl-2β,3aβ,6aβ-octahydrocyclopenta[b]-pyrrol-2-carbonsäure-benzylester

10 mMol S-Alanyl-2S,3aR,6aR-octahydrocyclopenta[b]pyrrol-2-carbonsäure-benzylester werden zusammen mit 10 mMol 3-Benzoyl-acrylsäureethylester und 10 mMol Triäthylamin in 100 ml wasserfreiem Äthanol gelöst und das Gemisch 24 Stunden bei 20 bis 25 °C gerührt. Man neutralisiert mit 1n Salzsäure, dampft zur Trockene ein und nimmt den Rückstand mit Essigester/Wasser auf. Die Essigesterphase wird getrocknet, eingedampft und an Kieselgel chromatographiert.

Beispiel 28

N-(1S-Carbethoxy-3-keto-3-phenyl-propyl)-S-alanyl-2S,3aS,7aR-octahydroindol-2-carbonsäure

Diese Verbindung wird analog der in Beispiel 27 b) beschriebenen Verfahrensweise ausgehend von N-(S-Alanyl)-2S,3aS,7aR-octahydroindol-2-carbonsäure-tert.butylester hergestellt. Die Abspaltung der tert.Butyl-Gruppe erfolgt mit Trifluoressigsäure.

$^1$H-NMR-Daten:

1,2 (d+t, 6H); 1,3–3,6 (m, 16H); 4,2 (q, 2H); 4,1–4,6 (m, 4H); 7,3–8,1 (m, 5H).

Beispiel 29

N-(1S-Carbethoxy-3-keto-3-phenyl-propyl)-S-alanyl-2S,3aR,6aS-octahydrocyclopenta[b]-pyrrol-2-carbonsäure

Diese Verbindung wird analog der in Beispiel 27 b) beschriebenen Verfahrensweise ausgehend von N-(S-Alanyl)-2S,3aR,6aS-octahydrocyclopenta[b]pyrrol-2-carbonsäure-tert.butylester hergestellt; die Abspaltung der tert.Butyl-Gruppe erfolgt mit Trifluoressigsäure.

$^1$H-NMR-Daten:

1,1 (d, 3H); 1,35 (t, 3H); 1,0–3,7 (m, 14H); 4,2 (q, 2H); 4,0–4,7 (m, 4H); 7,3–8,0 (m, 5H).

Beispiel 30

N-(1S-Carbethoxy-3-keto-3-phenyl-propyl)-S-alanyl-2S,3aS,6aR-octahydrocyclopenta[b]-pyrrol-2-carbonsäure

Diese Verbindung wird analog der in Beispiel 1 beschriebenen Verfahrensweise, gefolgt von einer Abspaltung der tert.Butylgruppe mit Trifluoressigsäure, hergestellt.

$^1$H-NMR-Daten:

1,1 (d, 3H); 1,35 (t, 3H); 1,0–3,8 (m, 14H); 4,2 (q, 2H); 4,0–4,7 (m, 4H); 7,2–8,1 (m, 5H).

Beispiel 31

N-(1S-Carbethoxy-3-keto-3-phenyl-propyl)-S-alanyl-2S,3aR,8aS-decahydrocyclohepta[b]-pyrrol-2-carbonsäure

Diese Verbindung wird analog der in Beispiel 1 beschriebenen Reaktionsweise, gefolgt von der Abspaltung der tertiär-Butylgruppe mit Trifluoressigsäure, hergestellt.

$^1$H-NMR-Daten:

1,2 (d, 3H); 1,4 (t, 3H); 1,2–3,8 (m, 18H); 4,1 (q, 2H); 4,0–4,6 (m, 4H); 7,2–8,2 (m, 5H).

Beispiel 32

N-(1S-Carbethoxy-3-keto-3-phenyl-propyl)-S-alanyl-2S,3aS,8aR-decahydrocyclohepta[b]-pyrrol-2-carbonsäure

Diese Verbindung wird analog der in Beispiel 1 beschriebenen Verfahrensweise, gefolgt von der Abspaltung der tert.Butylgruppe mit Trifluoressigsäure, hergestellt.

$^1$H-NMR-Daten:

1,2 (d+t, 6H); 1,2–3,8 (m, 18H); 4,2 (q, 2H); 4,0–4,6 (m, 4H); 7,1–8,1 (m, 5H).

Beispiel 33

a) N-(1S-Carbethoxy-3-hydroxy-3-phenyl-propyl)-S-alanyl-2S,3aR,7aS-octahydroindol-2-carbonsäure

Diese Verbindung wird analog der in Beispiel 33b) beschriebenen Verfahrensweise hergestellt.

$^1$H-NMR-Daten:

1,2 (d, 3H); 1,3 (t, 3H); 1,3–3,8 (m, 16H); 4,2 (q, 2H); 4,0–4,6 (m, 4H); 4,7 (d, 1H); 7,1–7,4 (m, 5H).

b) N-(1-S-Carbethoxy-3-phenyl-3-hydroxy-propyl)-S-alanyl-2β,3aβ,7aβ-octahydroindol-2-carbonsäure

1 g N-(1-S-Carbethoxy-3-phenyl-3-oxo-propyl)-S-alanyl-2β,3aβ,7aβ-octahydroindol-2-carbonsäure werden in 50 ml wasserfreiem Ethanol gelöst und mit 50 mg Palladium/Kohle bei 20 bis 25 °C unter Normaldruck mit 1 Mol Äquivalent Wasserstoff hydriert. Nach Abfiltrieren des Katalysators wird die Lösung eingedampft, der Rückstand mit Diisopropyläther verrieben und abgesaugt. Ausbeute: 0,7 g.

$^1$H-NMR-Daten:

1,2 (d, 3H); 1,3 (t, 3H); 1,3–3,8 (m, 16H); 4,2 (q, 2H); 4,1–4,6 (m, 4H); 4,7 (d, 1H); 7,1–7,4 (m, 5H).

Beispiel 34

N-(1S-Carbethoxy-3-hydroxy-3-phenyl-propyl)-S-alanyl-2S,3aS,6aR-octahydrocyclopenta[b]pyrrol-2-carbonsäure

Diese Verbindung wird analog der in Beispiel 33 b) beschriebenen Verfahrensweise hergestellt.
$^1$H-NMR-Daten:
1,2 (d+t, 3H); 1,1–3,9 (m, 14H); 4,2 (q, 2H); 4,0–4,7 (m, 4H); 4,8 (d, 1H); 7,1–7,4 (m, 5H).

Beispiele 35 bis 48:
Die in diesen Beispielen genannten Verbindungen werden analog der in nachstehendem Beispiel beschriebenen Verfahrensweise hergestellt.

N-(1-S-Carboxy-3-phenyl-propyl)-S-alanyl-2S,3aR,7aR-octahydroindol-2-carbonsäure
Eine Lösung von 1 g N-(1-S-Carbethoxy-3-phenyl-propyl)-S-alanyl-2S,3aR,7aR-octahydroindol-2-carbonsäure-Hydrochlorid in 10 ml Wasser wird mit zwei Äquivalenten Kaliumhydroxid und einem 10%igen Überschuss 4n-Kaliumhydroxid versetzt. Nach 8stündigem Rühren bei 20 bis 25 °C wird die Reaktionslösung mit 2n-Salzsäure auf einen pH-Wert von 4,0 gestellt und im Vakuum eingeengt. Der Rückstand wird in Essigester aufgenommen; von abgeschiedenem Salz wird abfiltriert. Die Essigesterlösung wird eingeengt, der Rückstand mit Diisopropylether verrieben und abgesaugt. Ausbeute: 0,6 g
$^1$H-NMR-Daten:
1,2 (d, 3H); 1,2–3,8 (m, 18H); 4,0–4,6 (m, 4H); 7,2 (s, 5H).

Beispiel 35
N-(1S-Carboxy-3-phenyl-propyl)-S-alanyl-2S,3aR,7aS-octahydroindol-2-carbonsäure
$^1$H-NMR-Daten:
1,2 (d, 3H); 1,2–3,8 (m, 18H); 4,0–4,6 (m, 4H); 7,2 (s, 5H).

Beispiel 36
N-(1S-Carboxy-3-phenyl-propyl)-S-alanyl-2S,3aS,7aR-octahydroindol-2-carbonsäure
$^1$H-NMR-Spektrum:
1,2 (d, 3H); 1,2–3,8 (m, 18H); 4,0–4,6 (m, 4H); 7,2 (s, 5H).

Beispiel 37
N-(1S-Carboxy-3-phenyl-propyl)-S-alanyl-2S,3aR,6aS-octahydrocyclopenta[b]pyrrol-2-carbonsäure
$^1$H-NMR-Daten:
1,2 (d, 3H); 1,1–3,7 (m, 16H); 4,0–4,6 (m, 4H); 7,2 (s, 5H).

Beispiel 38
N-(1S-Carboxy-3-phenyl-propyl)-S-alanyl-2S,3aS,6aR-octahydro-cyclopenta[b]pyrrol-2-carbonsäure
$^1$H-NMR-Spektrum:
1,2 (d, 3H); 1,1–3,8 (m, 16H); 4,0–4,6 (m, 4H); 7,2 (s, 5H).

Beispiel 39
N-(1S-Carboxy-3-phenyl-propyl)-S-alanyl-2S,3aR,8aS-decahydrocyclohepta[b]pyrrol-2-carbonsäure
$^1$H-NMR-Daten:
1,2 (d, 3H); 0,9–3,6 (m, 20H); 4,0–4,6 (m, 4H); 7,2 (s, 5H).

Beispiel 40
N-(1S-Carboxy-3-phenyl-propyl)-S-alanyl-2S,3aS,8aR-decahydrocyclohepta[b]pyrrol-2-carbonsäure
$^1$H-NMR-Daten:
1,2 (d, 3H); 0,8–3,6 (m, 20H); 4,0–4,6 (m, 4H); 7,2 (s, 5H).

Beispiel 41
N-(1S-Carboxy-3-keto-3-phenyl-propyl)-S-alanyl-2S,3aR,7aS-octahydroindol-2-carbonsäure
$^1$H-NMR-Daten:
1,2 (d, 3H); 0,9–3,6 (m, 16H); 3,9–4,7 (m, 4H); 7,1–8,2 (m, 5H).

Beispiel 42
N-(1S-Carboxy-3-keto-3-phenyl-propyl)-S-alanyl-2S,3aS,7aR-octahydroindol-2-carbonsäure
$^1$H-NMR-Daten:
1,2 (d, 3H); 0,9–3,6 (m, 16H); 3,9–4,7 (m, 4H); 7,2–8,1 (m, 5H).

Beispiel 43
N-(1S-Carboxy-3-keto-3-phenyl-propyl)-S-alanyl-2S,3aR,6aS-octahydrocyclopenta[b]pyrrol-2-carbonsäure
$^1$H-NMR-Daten:
1,2 (d, 3H); 1,1–3,7 (m, 14H); 3,9–4,6 (m, 4H); 7,2–8,1 (m, 5H).

Beispiel 44
N-(1S-Carboxy-3-keto-3-phenyl-propyl)-S-alanyl-2S,3aS,6aR-octahydrocyclopenta[b]pyrrol-2-carbonsäure
$^1$H-NMR-Daten:
1,2 (d, 3H); 1,1–3,7 (m, 14H); 3,9–4,6 (m, 4H); 7,2–8,1 (m, 5H).

Beispiel 45
N-(1S-Carboxy-3-keto-3-phenyl-propyl)-S-alanyl-2S,3aR,8aS-decahydrocyclohepta[b]pyrrol-2-carbonsäure
$^1$H-NMR-Daten:
1,2 (d, 3H); 1,1–3,7 (m, 18H); 3,9–4,6 (m, 4H); 7,3–8,2 (m, 5H).

Beispiel 46
N-(1S-Carboxy-3-keto-3-phenyl)-alanyl-2S,3aS,8aR-decahydrocyclohepta[b]pyrrol-2-carbonsäure
$^1$H-NMR-Daten:
1,2 (d, 3H); 1,1–3,7 (m, 18H); 3,9–4,6 (m, 4H); 7,3–8,2 (m, 5H).

Beispiel 47
N-(1S-Carboxy-3-hydroxy-3-phenyl-propyl)-S-alanyl-2S,3aR,7aS-octahydroindol-2-carbonsäure
$^1$H-NMR-Spektrum:
1,2 (d, 3H); 1,1–3,7 (m, 16H); 3,8–4,6 (m, 4H); 4,7 (m, 1H); 7,1–7,4 (m, 5H).

Beispiel 48
N-(1S-Carboxy-3-hydroxy-3-phenyl-propyl)-S-alanyl-2S,3aS,6aR-octahydrocyclopenta[b]-pyrrol-2-carbonsäure
$^1$H-NMR-Daten:
1,2 (d, 3H); 1,1–3,7 (m, 14H); 3,9–4,5 (m, 4H); 4,7 (d, 1H); 7,1–7,4 (m, 5H).

Beispiel 49
2β,3aβ,7aα-Octahydroindol-2-carbonsäure

290 g des nach Beispiel 50 erhaltenen Acylamino-Derivates werden wie in Beispiel 51 angegeben mit 2n Salzsäure erhitzt. Man engt im Vakuum ein, nimmt in 1 l Isopropanol auf und reduziert mit etwa 35 g $NaBH_4$, das portionsweise in 30 Minuten zugesetzt wird. Die Reaktionstemperatur sollte 40–50 °C betragen. Man lässt ca. 4 Stunden reagieren, engt im Vakuum ein, stellt mit verdünnter Salzsäure auf pH 6,5, sättigt mit festem Natriumchlorid und extrahiert die Aminosäuren mehrfach mit n-Butanol. Der nach Einengen der organischen Phase hinterbleibende Rückstand wird aus Chloroform/Diisopropyläther wie im Beispiel 51 beschrieben, fraktioniert kristallisiert.

Ausbeuten: 40–60 g 2β,3aα,7aα-Produkt
20 g Mischfraktion
100–130 g trans-Produkt.

In gleicher Weise lassen sich 2β,3aβ,7aα-2-Azabicyclo-(5.3.0)-decan-3-carbonsäure und 2β,3aβ,7aα-2-Azabicyclo-(6.3.0)undecan-3-carbonsäure herstellen.

Beispiel 50
2β,3aβ,7aβ-Octahydroindol-2-carbonsäure

a) N-Acetyl-3aβ,7aβ-Octahydroindol

Zu einer Lösung von 77 g Indol in 700 ml Eisessig werden 3,5 g Platinoxid gegeben. Man hydriert zunächst 16 Stunden bei 20 bis 25 °C unter 100 Atmosphären und anschliessend bei 20 bis 25 °C unter Normaldruck, bis die Wasserstoffaufnahme beendet ist. Der Katalysator wird abgesaugt und das Lösungsmittel im Vakuum abdestilliert. Der Rückstand wird in Wasser aufgenommen und mit gesättigter Kaliumcarbonatlösung alkalisch gestellt. Nach Sättigung mit Natriumchlorid wird die wässrige Phase vier mal mit Methylenchlorid extrahiert und die organische Phase getrocknet und eingedampft.

Der Rückstand wird in 250 ml Pyridin aufgenommen, mit 93 ml Acetanhydrid versetzt, wonach man das Gemisch 12 Stunden bei 20 bis 25 °C reagieren lässt. Nach Abdestillieren des Pyridin wird der Rückstand mit Wasser versetzt und mit konz. wässr. Natriumhydroxid alkalisch gestellt. Man extrahiert die wässrige Phase mit Methylenchlorid, wäscht die erhaltene organische Phase mit 2n-Salzsäure und anschliessend mit Wasser. Nach Trocknen und Einengen der Lösung wird der Rückstand destilliert. Ausbeute: 85 g; Kp: 91 bis 95 °C/0.2 mm Hg

b) N-Acetyl-2-methoxy-3aβ,7aβ-octahydroindol

54 g N-Acetyl-3aβ,7aβ-octahydroindol werden in Methanol unter Zusatz von Tetramethylammoniumtetrafluoroborat gemäss den Angaben in Liebigs Ann. Chem. 1978, Seite 1719 anodisch oxidiert. Das Lösungsmittel wird abdestilliert und der Rückstand über 500 g Kieselgel mittels Essigester filtriert. Aus der Essigester-Lösung werden nach dem Eindampfen 53,6 g des obengenannten Produktes erhalten. $R_f$-Wert (Dünnschichtchrom.): 0.33 (Kieselgel, Essigester).

c) N-Acetyl-2β,3aβ,7aβ-octahydroindol-2-carbonsäurenitril

Zu einer Lösung von 49,8 g N-Acetyl-2-methoxy-3aβ,7aβ-octahydroindol in 250 ml Methylenchlorid werden bei −40 °C 25 g Trimethylsilylcyanid in 50 ml Methylenchlorid zugetropft. Anschliessend werden 35,9 g Bortrifluoridätherat so zugetropft, dass die Temperatur des Reaktionsgemisches −20 °C nicht übersteigt. Nach 2 Stunden Reaktionszeit bei −20 °C erhöht man die Temperatur langsam auf 0 °C, rührt über Nacht bei 0 °C und anschliessend noch eine Stunde bei 20 bis 25 °C. Man gibt Wasser hinzu und rührt 10 Min. Die wässrige Phase wird dreimal mit Methylenchlorid extrahiert. Die vereinigten organischen Extrakte werden getrocknet, eingeengt und der Rückstand mit Diisopropyläther verrieben. Ausbeute: 47 g; Fp.: 128° bis 130 °C.

d) 2β,3aβ,7aβ-Octahydroindol-2-carbonsäure

10 g N-Acetyl-2β,3aβ,7aβ-octahydroindol-2-carbonsäurenitril werden in 30 ml konz. Bromwasserstoff zwei Stunden zum Sieden erhitzt. Nach Abdestillieren des Bromwasserstoffs wird der Rückstand mit wenig Aceton verrührt und abgesaugt.

Eine wässrige Lösung des Produktes wird mit einem schwach basischen Ionenaustauscher auf einen pH-Wert von 6,0 gestellt. Nach Filtration wird die Lösung eingedampft und der Rückstand über Kieselgel mit einem Gemisch von Methylenchlorid, Methanol, Eisessig und Wasser im Verhältnis 20:10:0,5:0,5 filtriert. Das Eluat wird eingeengt und der Rückstand mit Diisopropyläther verrieben. Ausbeute: 7,6 g
$^1$H-NMR-Spektrum*:
1,0–2,5 (m, 11H); 3,4–3,9 (m, 1H); 4,0–4,5 (m, 1H); 7,5–8,3 (s-breit, mit $D_2O$ austauschbar)

Verfährt man analog den unter Beispiel 50 beschriebenen Verfahrensweisen, so erhält man die unter Beispiel 51 im folgenden genannten Verbindungen:

Beispiel 51
2β,3aβ,6aβ-Octahydrocyclopenta[b]-pyrrol-2-carbonsäure

a) N-Acetyl-cis-octahydrocyclopenta[b]-pyrrol
$^1$H-NMR-Daten:
1,0–2,3 (m, 9H); 2,0 (d, 3H); 3,3 bis 4,2 (m, 3H).

* Die $^1$H-NMR-Daten wurden hier wie in den übrigen Beispielen in $CDCl_3$ ermittelt und sind in ppm angegeben.

b) N-Acetyl-2-methoxy-cis-octahydrocyclopenta[b]-pyrrol
$^1$H-NMR-Daten:
0,9–2,6 (m, 9H); 2,1 (s, 3H); 3,3 (s, 3H); 3,8–4,3 (m, 1H); 4,7–5,5 (m, 1H).

c) N-Acetyl-2-cyano-cis-octahydrocyclopenta-[b]pyrrol
$^1$H-NMR-Daten:
1,0–3,0 (m, 9H); 2,1 (d, 3H); 3,5–4,1 (m, 1H); 4,4–4,7 (m, 1H).

d) 2β,3aβ,6aβ-Octahydrocyclopenta[b]pyrrol-2-carbonsäure
$^1$H-NMR-Daten:
1,0–2,3 (m, 9H); 3,5–3,9 (m, 1H); 4,0–4,6 (m, 1H); 7,7–8,4 (s-breit, mit $D_2O$ austauschbar).

**Patentansprüche für die Vertragsstaaten: BE, CH, DE, FR, GB, IT, LI, LU, NL, SE**

1. Verbindung der Formel I,

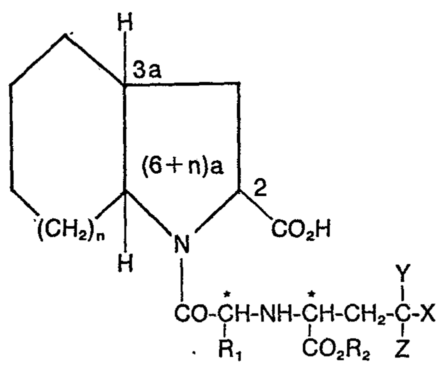

I

in der die Wasserstoffatome an den Brückenkopf-C-Atomen 3 a und (6+n)a zueinander trans-konfiguriert sind, und worin
n = 0, 1 oder 2,
$R_1$ = Methyl,
$R_2$ = Wasserstoff, ($C_1$–$C_4$)-Alkyl oder Benzyl,
Y = Wasserstoff oder Hydroxy,
Z = Wasserstoff oder
Y und Z = zusammen Sauerstoff und
X = Phenyl bedeuten,
sowie deren physiologisch unbedenklichen Salze.

2. Verbindung der Formel I gemäss Anspruch 1, dadurch gekennzeichnet, dass das C-Atom in Position 2 des bicyclischen Ringsystems sowie die mit einem Stern markierten C-Atome der Seitenkette S-Konfiguration aufweisen, sowie deren physiologisch unbedenklichen Salze.

3. Verbindung der Formel I gemäss Anspruch 1 oder 2, dadurch gekennzeichnet, dass
$R_2$ = Wasserstoff oder Ethyl bedeutet, sowie deren physiologisch unbedenklichen Salze.

4. N-(1-S-Carbethoxy-3-phenyl-propyl)-S-alanyl-2S,3aR,7aS-octahydroindol-2-carbonsäure,
sowie deren physiologisch unbedenklichen Salze.

5. N-(1-S-Carbethoxy-3-phenyl-propyl)-S-alanyl-2S,3aS,7aR-octahydroindol-2-carbonsäure,
sowie deren physiologisch unbedenklichen Salze.

6. Verfahren zur Herstellung der Verbindungen der Formel I gemäss einem der Ansprüche 1 bis 5, dadurch gekennzeichnet,
a) dass man eine Verbindung der Formel II,

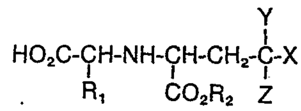

II

worin $R_1$, $R_2$, X, Y und Z die Bedeutungen wie in Formel I haben, mit einer Verbindung der Formel III,

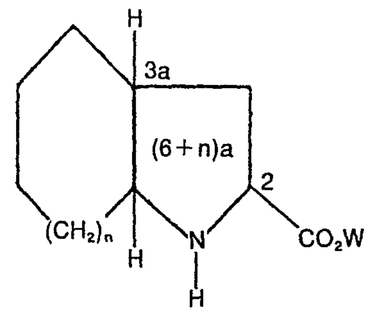

III

in der die H-Atome an den C-Atomen 3 a und (6+n)a zueinander trans-konfiguriert sind,
n = 0, 1 oder 2 und
W = einen hydrogenolytisch oder sauer abspaltbaren Rest bedeuten, umsetzt und anschliessend den Rest W und gegebenenfalls den Rest $R_2$ unter Bildung der freien Carboxygruppen abspaltet, oder,
b) dass man zur Herstellung der Verbindungen der Formel I, in der Y und Z zusammen Sauerstoff bedeuten,
$b_1$) eine Verbindung der Formel IV, in der die H-Atome an den C-Atomen 3 a und (6+n)a

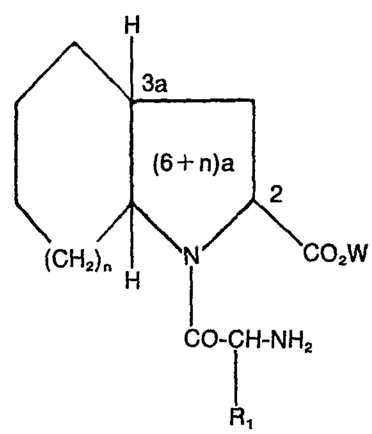

IV

zueinander trans-konfiguriert sind und worin n und $R_1$ die Bedeutungen wie in Formel I und W die Bedeutung wie in Formel III besitzen, mit einer Verbindung der Formel V,

$$R_2O_2C\text{–}CH = CH\text{–}CO\text{–}X \qquad V$$

worin $R_2$ und X die Bedeutungen wie in Formel I haben, umsetzt und anschliessend den Rest W

und gegebenenfalls auch den Rest $R_2$ unter Bildung der freien Carboxygruppen abspaltet, oder

$b_2$) eine Verbindung der unter $b_1$) genannten Formel IV mit einer Verbindung der allgemeinen Formel VI, worin $R_2$ die Bedeutung wie in Formel I hat, und mit einer Verbindung der allgemeinen Formel VII,

$OHC{-}CO_2R_2$ $X{-}CO{-}CH_3$

VI VII

worin X die Bedeutung wie in Formel I hat, umsetzt und anschliessend den Rest W und gegebenenfalls den Rest $R_2$ unter Bildung der freien Carboxygruppen abspaltet, oder

c) dass man zur Herstellung von Verbindungen der Formel I, in der Y und Z jeweils Wasserstoff bedeuten,

$c_1$) eine Verbindung der unter $b_1$) genannten Formel IV mit einer Verbindung der Formel VIII,

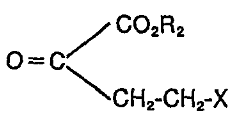

VIII

worin $R_2$ und X die Bedeutungen wie in Formel I besitzen, umsetzt, die erhaltenen Schiff-Basen reduziert und anschliessend den Rest W und gegebenenfalls den Rest $R_2$ unter Bildung der freien Carboxygruppen abspaltet, oder

$c_2$) eine Verbindung der Formel I, in der Y und Z zusammen Sauerstoff bedeuten, katalytisch mit Wasserstoff reduziert, oder

d) dass man zur Herstellung von Verbindungen der Formel I, in der Y = Hydroxy und Z = Wasserstoff bedeuten, eine Verbindung der Formel I, in der Y und Z zusammen Sauerstoff bedeuten, katalytisch mittels Wasserstoff oder mit einem Reduktionsmittel wie Natriumborhydrid reduziert,

und die nach (a)–(d) erhaltenen Verbindungen gegebenenfalls in ihre physiologisch verträglichen Salze überführt.

7. Verbindung gemäss einem der Ansprüche 1 bis 5 zur Anwendung als Heilmittel.

8. Verbindung gemäss Anspruch 7 zur Anwendung als Heilmittel bei der Behandlung des Bluthochdrucks.

9. Verbindung gemäss Anspruch 7 oder 8 zur Anwendung in Kombination mit einem Diuretikum.

10. Verbindung der Formel III′,

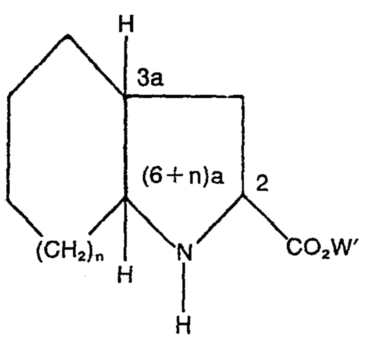

III′

in der die H-Atome an den C-Atomen 3 a und (6+n)a zueinander trans-konfiguriert sind und worin

$n = 0$, 1 oder 2

W′ = Wasserstoff, ($C_1$–$C_{18}$)-Alkyl oder ($C_7$–$C_{10}$)-Aralkyl bedeuten.

11. Verfahren zur Herstellung der Verbindungen der Formeln III′ gemäss Anspruch 10, dadurch gekennzeichnet,

a) dass man eine Verbindung der Formel IX,

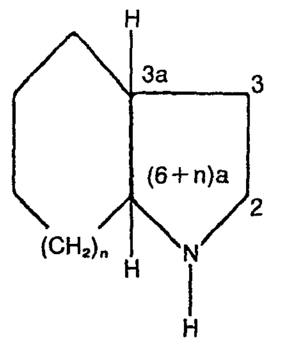

IX

worin die H-Atome an den C-Atomen 3 a und (6+n)a zueinander trans-konfiguriert sind und worin n die Zahl 0, 1 oder 2 bedeutet, acyliert, anschliessend mit einem aliphatischen Alkohol in Gegenwart eines Leitsalzes zu einer Verbindung der Formel X,

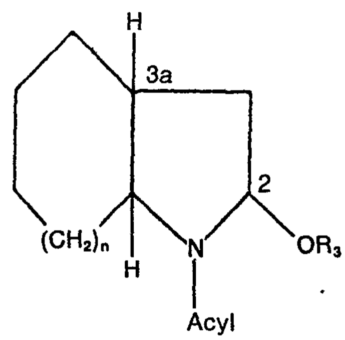

X

worin n die oben genannte Bedeutung hat, Acyl ein aliphatischer oder aromatischer Acylrest ist und $R_3$ ($C_1$–$C_4$)-Alkyl bedeutet, anodisch oxydiert, diese mit Trimethylsilylcyanid in einem aprotischen organischen Lösemittel in Gegenwart einer Lewis-Säure zu einer Verbindung der Formel XI,

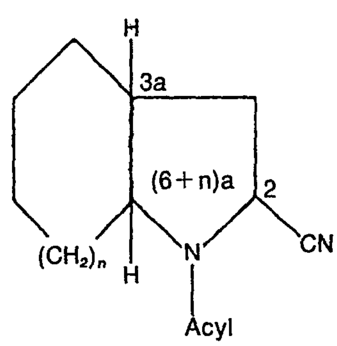

XI

worin die H-Atome an den C-Atomen 3 a und (6+n)a zueinander trans-konfiguriert sind und worin n die oben genannte Bedeutung hat,

und worin n die oben genannte Bedeutung hat, umsetzt und diese durch Einwirkung von Säuren oder Basen zu einer Verbindung der Formel III' mit W' = Wasserstoff hydrolysiert und letztere gegebenenfalls verestert, oder

b) dass man eine Verbindung der Formel XII,

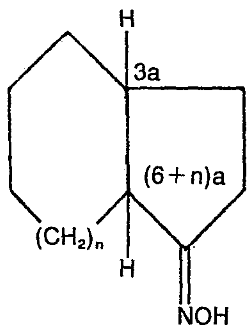

XII

worin die H-Atome an den C-Atomen 3 a und (6 + n)a trans-konfiguriert sind und n die oben genannte Bedeutung besitzt in einer Beckmann-Umlagerung zu einer Verbindung der Formel XIII

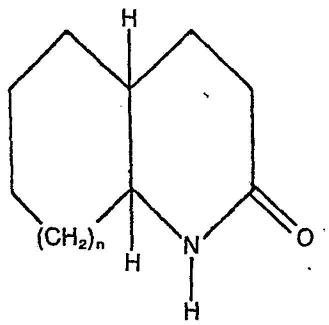

XIII

worin n die obengenannte Bedeutung besitzt, umsetzt, diese zu einer Verbindung der Formel XIV,

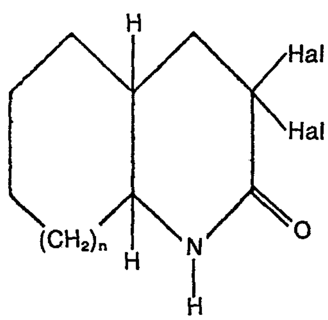

XIV

worin n die obengenannte Bedeutung besitzt und Hal ein Halogenatom bedeutet, halogeniert, diese zu einer Verbindung der Formel XV,

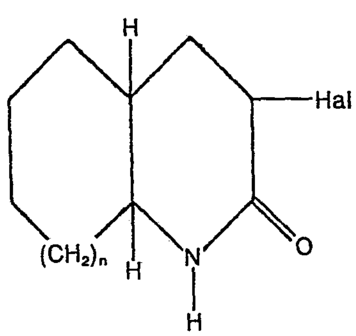

XV

worin n und Hal die oben genannten Bedeutungen besitzen, in einem polaren protischen Lösungsmittel unter Zusatz eines Säureakzeptors katalytisch reduziert, und diese anschliessend in einem alkoholischen Lösungsmittel unter Einwirkung einer Base zu einer Verbindung der Formel III' mit W' = Wasserstoff umsetzt und diese gegebenenfalls verestert, oder

c) dass man eine Verbindung der Formel XVI,

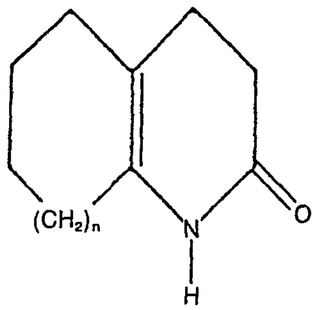

XVI

worin n die oben genannte Bedeutung besitzt mit Natriumformiat und Ameisensäure zu einer Verbindung der unter b) genannten Formel XIII reduziert, und diese, wie unter b) beschrieben, weiter umsetzt, oder

d) dass man Verbindungen der Formel XXIa oder b, in welcher n vorstehende Bedeutung hat,

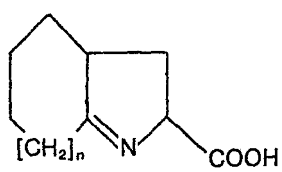

XXIa

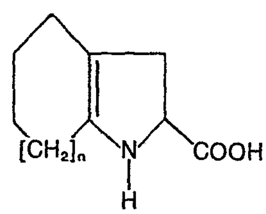

XXIb

gegebenenfalls nach Überführung in ihre $C_1$–$C_{18}$-Alkyl- oder $C_7$–$C_{10}$-Aralkylester durch katalytische Hydrierung in Gegenwart von Übergangsmetallkatalysatoren oder durch Reduktion mit Boran-Amin-Komplexen oder komplexen Borhydriden in Verbindungen der Formel III', in welcher W' für Wasserstoff, Alkyl mit 1 bis 18 C-Atomen oder Aralkyl mit 7 bis 10 C-Atomen steht, überführt, gegebenenfalls erhaltene Ester der Formel III' verseift und, falls W' Wasserstoff ist, gegebenenfalls zu Verbindungen der Formel III' worin W' für Alkyl mit 1 bis 18 C-Atomen oder Aralkyl mit 7 bis 10 C-Atomen steht, verestert.

12. Pharmazeutisches Mittel enthaltend eine Verbindung gemäss einem der Ansprüche 1 bis 5.

13. Mittel gemäss Anspruch 12, dadurch gekennzeichnet, dass es zusätzlich ein Diuretikum enthält.

**Patentansprüche für den Vertragsstaat: Österreich**

1. Verfahren zur Herstellung der Verbindungen der Formel I,

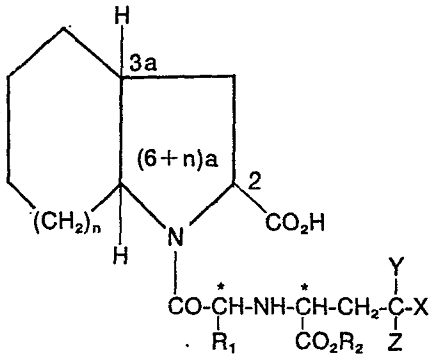

I

in der die Wasserstoffatome an den Brückenkopf-C-Atomen 3a und (6+n)a zueinander trans-konfiguriert sind und worin

n = 0, 1 oder 2,
$R_1$ = Methyl,
$R_2$ = Wasserstoff, ($C_1$–$C_4$)-Alkyl oder Benzyl,
Y = Wasserstoff oder Hydroxy,
Z = Wasserstoff oder
Y und Z = zusammen Sauerstoff und
X = Phenyl bedeuten, sowie

deren physiologisch unbedenklichen Salzen, dadurch gekennzeichnet,

a) dass man eine Verbindung der Formel II,

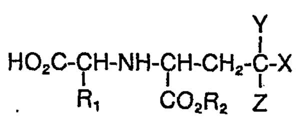

II

worin $R_1$, $R_2$, X, Y und Z die Bedeutungen wie in Formel I haben, mit einer Verbindung der Formel III

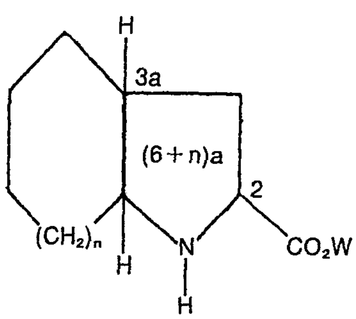

III

in der die H-Atome an den C-Atomen 3 a und (6+n)a zueinander trans-konfiguriert sind und wobei

n = 0, 1 oder 2 und
W = einen hydrogenolytisch oder sauer abspaltbaren Rest bedeuten, umsetzt und anschliessend den Rest W und gegebenenfalls den Rest $R_2$ unter Bildung der freien Carboxygruppen abspaltet, oder,

b) dass man zur Herstellung der Verbindungen der Formel I, in der Y und Z zusammen Sauerstoff bedeuten,

$b_1$) eine Verbindung der Formel IV,

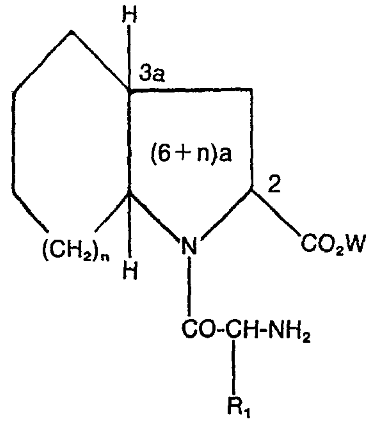

IV

in der die H-Atome an den C-Atomen 3a und (6+n)a zueinander trans-konfiguriert sind und worin n und $R_1$ die Bedeutungen wie in Formel I und W die Bedeutung wie in Formel III besitzen, mit einer Verbindung der Formel V,

$$R_2O_2C{-}CH = CH{-}CO{-}X \qquad V$$

worin $R_2$ und X die Bedeutungen wie in Formel I haben, umsetzt und anschliessend den Rest W und gegebenenfalls auch den Rest $R_2$ unter Bildung der freien Carboxygruppen abspaltet, oder

$b_2$) eine Verbindung der unter $b_1$) genannten Formel IV mit einer Verbindung der allgemeinen Formel VI, worin $R_2$ die Bedeutung wie in Formel I hat, und mit einer Verbindung der allgemeinen Formel VII,

$OHC{-}CO_2R_2$ (VI) $\qquad$ $X{-}CO{-}CH_3$ (VII)

worin X die Bedeutung wie in Formel I hat, umsetzt und anschliessend den Rest W und gegebenenfalls den Rest $R_2$ unter Bildung der freien Carboxygruppen abspaltet, oder

c) dass man zur Herstellung von Verbindungen der Formel I, in der Y und Z jeweils Wasserstoff bedeuten,

$c_1$) eine Verbindung der unter $b_1$) genannten Formel IV mit einer Verbindung der Formel VIII,

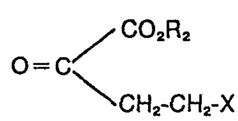

VIII

worin $R_2$ und X die Bedeutungen wie in Formel I besitzen, umsetzt, die erhaltenen Schiff-Basen reduziert und anschliessend den Rest W und gegebenenfalls den Rest $R_2$ unter Bildung der freien Carboxygruppen abspaltet, oder

$c_2$) eine Verbindung der Formel I, in der Y und Z zusammen Sauerstoff bedeuten, katalytisch mit Wasserstoff reduziert, oder

d) dass man zur Herstellung von Verbindungen der Formel I, in der Y = Hydroxy und Z = Wasserstoff bedeuten, eine Verbindung der Formel I, in der Y und Z zusammen Sauerstoff bedeuten, katalytisch mittels Wasserstoff oder mit einem Reduktionsmittel wie Natriumborhydrid reduziert, und die nach (a)–(d) erhaltenen Verbindungen gegebenenfalls in ihre physiologisch verträglichen Salze überführt.

2. Verfahren zur Herstellung von Verbindungen der Formel I gemäss Anspruch 1, dadurch gekennzeichnet, dass das C-Atom in Position 2 des bicyclischen Ringsystems sowie die mit einem Stern markierten C-Atome der Seitenkette S-Konfiguration aufweisen.

3. Verfahren zur Herstellung von Verbindungen der Formel I gemäss Anspruch 1 oder 2, dadurch gekennzeichnet, dass $R_2$ = Wasserstoff oder Ethyl bedeutet.

4. Verfahren gemäss einem der Ansprüche 1 bis 3, dadurch gekennzeichnet, dass
N-(2-S-Carbethoxy-3-phenyl-propyl)-S-alanyl-2S, 3aR,7aS-octahydroindol-2-carbonsäure
oder deren physiologisch verträgliches Salz hergestellt wird.

5. Verfahren gemäss einem der Ansprüche 1 bis 3, dadurch gekennzeichnet, dass
N-(1-S-Carbethoxy-3-phenyl-propyl)-S-alanyl-2S, 3aS,7aR-octahydroindol-2-carbonsäure
oder deren physiologisch verträgliches Salz hergestellt wird.

6. Verfahren zur Herstellung der Verbindungen der Formel III'

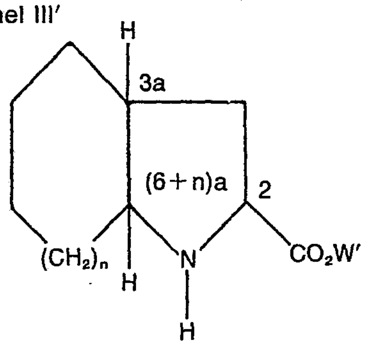

III'

in der die H-Atome an den C-Atomen 3 a und (6+n)a zueinander trans-konfiguriert sind und worin

n = 0, 1 oder 2

W' = Wasserstoff, ($C_1$–$C_{18}$)-Alkyl oder ($C_7$–$C_{10}$)-Aralkyl bedeutet,

dadurch gekennzeichnet,

a) dass man eine Verbindung der Formel IX,

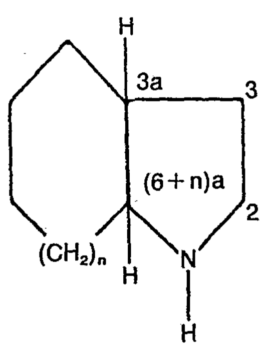

IX

worin die H-Atome an den C-Atomen 3 a und (6+n)a zueinander trans-konfiguriert sind und worin n die Zahl 0, 1 oder 2 bedeutet, acyliert, anschliessend mit einem aliphatischen Alkohol in Gegenwart eines Leitsalzes zu einer Verbindung der Formel X,

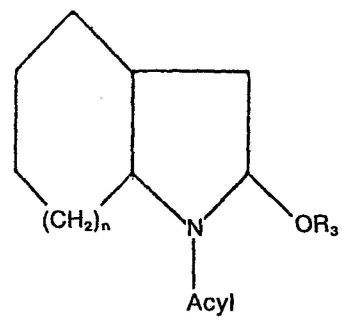

X

worin n die oben genannte Bedeutung hat, Acyl ein aliphatischer oder aromatischer Acylrest ist und $R_3$ ($C_1$–$C_4$)-Alkyl bedeutet, anodisch oxydiert, diese mit Trimethylsilylcyanid in einem aprotischen organischen Lösemittel in Gegenwart einer Lewis-Säure zu einer Verbindung der Formel XI,

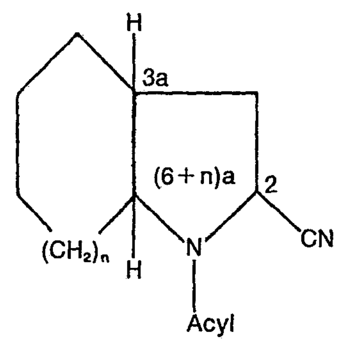

XI

worin die H-Atome an den C-Atomen 3 a und (6+n)a zueinander trans-konfiguriert sind und worin n die oben genannte Bedeutung hat, umsetzt und diese durch Einwirkung von Säuren oder Basen zu einer Verbindung der Formel III' mit W' = Wasserstoff hydrolysiert und letztere gegebenenfalls verestert, oder

b) dass man eine Verbindung der Formel XII,

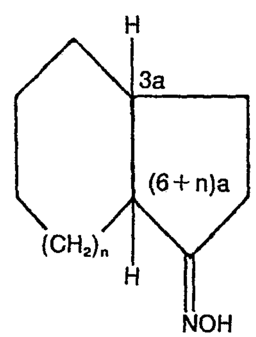

XII

worin die H-Atome an den C-Atomen 3 a und (6+n)a trans-konfiguriert sind und n die oben genannte Bedeutung besitzt in einer Beckmann-Umlagerung zu einer Verbindung der Formel XIII

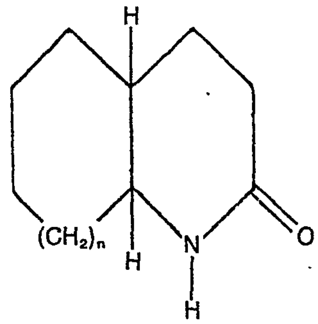

XIII

worin n die obengenannte Bedeutung besitzt, umsetzt, diese zu einer Verbindung der Formel XIV,

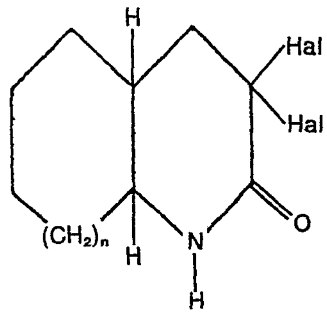

XIV

worin n die obengenannte Bedeutung besitzt und Hal ein Halogenatom bedeutet, halogeniert, diese zu einer Verbindung der Formel XV,

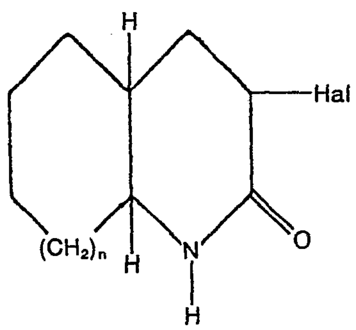

XV

worin n und Hal die oben genannten Bedeutungen besitzen, in einem polaren protischen Lösungsmittel unter Zusatz eines Säureakzeptors katalytisch reduziert, und diese anschliessend unter Einwirkung einer Base in einem alkoholischen Lösungsmittel zu einer Verbindung der Formel III′ mit W′ = Wasserstoff umsetzt und diese gegebenenfalls verestert, oder

c) dass man eine Verbindung der Formel XVI,

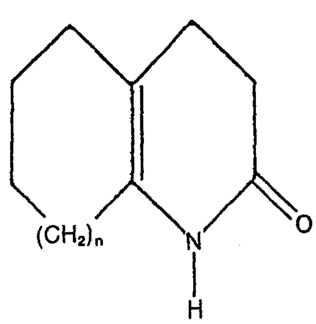

XVI

worin n die obengenannte Bedeutung besitzt mit Natriumformiat und Ameisensäure zu einer Verbindung der unter b) genannten Formel XIII reduziert, und diese, wie unter b) beschrieben, weiter umsetzt, oder

d) dass man Verbindungen der Formel XXIa oder b, in welcher n vorstehende Bedeutung hat,

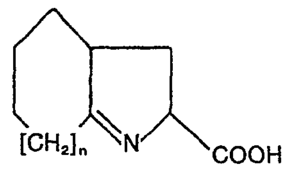

XXIa

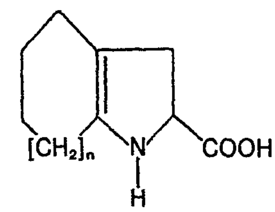

XXIb

gegebenenfalls nach Überführung in ihre $C_1$–$C_{18}$-Alkyl- oder $C_7$–$C_{10}$-Aralkylester durch katalytische Hydrierung in Gegenwart von Übergangsmetallkatalysatoren oder durch Reduktion mit Boran-Amin-Komplexen oder komplexen Borhydriden in Verbindungen der Formel III′ in welcher

W′ für Wasserstoff, Alkyl mit 1 bis 18 C-Atomen oder Aralkyl mit 7 bis 10 C-Atomen steht, überführt, gegebenenfalls erhaltene Ester der Formel III′ verseift und, falls W′ Wasserstoff ist, gegebenenfalls zu Verbindungen der Formel III′, worin W′ für Alkyl mit 1 bis 18 C-Atomen oder Aralkyl mit 7 bis 10 C-Atomen steht, verestert.

7. Verfahren zur Herstellung eines pharmazeutischen Mittels enthaltend eine Verbindung der Formel I gemäss einem der Ansprüche 1 bis 5, dadurch gekennzeichnet, dass man diese in eine geeignete Darreichungsform bringt.

8. Verfahren gemäss Anspruch 7, dadurch gekennzeichnet, dass man ein Diuretikum zusetzt.

**Claims for the Contracting states: BE, CH, DE, FR, GB, IT, LI, LU, NL, SE**

1. A compound of the formula I

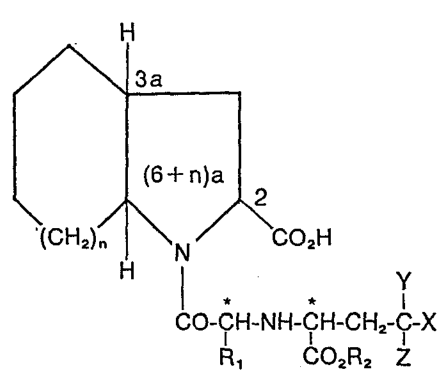

I

in which the hydrogen atoms on the bridgehead C atoms 3a and (6+n)a have the trans configuration relative to one another and wherein

n denotes 0, 1 or 2,
$R_1$ denotes methyl,
$R_2$ denotes hydrogen, $(C_1–C_4)$-alkyl or benzyl,
Y denotes hydrogen or hydroxyl,
Z denotes hydrogen or
Y and Z together denote oxygen and
X denotes phenyl,

and its physiologically acceptable salts.

2. A compound of the formula I as claimed in claim 1, characterized in that the C atom in position 2 of the bicyclic ring system and the C atom in the side chain labeled with an asterisk have the S configuration, and its physiologically acceptable salts.

3. A compound as claimed in claim 1 or 2, characterized in that $R_2$ denotes hydrogen or ethyl, and its physiologically acceptable salts.

4. N-(1-S-Carboethoxy-3-phenyl-propyl)-S-alanyl-2S,3aR,7aS-octahydroindole-2-carboxylic acid,
and its physiologically acceptable salts.

5. N-(1-S-Carboethoxy-3-phenyl-propyl)-S-alanyl-2S,3aS,7aR-octahydroindole-2-carboxylic acid,
and its physiologically acceptable salts.

6. A process for the preparation of the compounds of the formula I as claimed in any of claims 1 to 5, which

a) comprises reacting a compound of the formula II

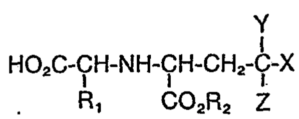 II

wherein $R_1$, $R_2$, X, Y and Z have the meanings as in formula I, with a compound of the formula III

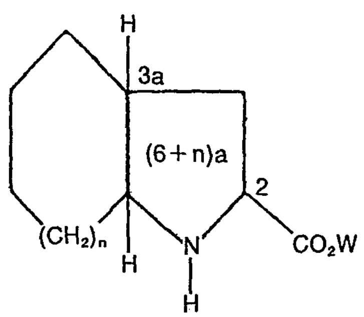 III

in which the H atoms on the C atoms 3a and (6+n)a have the trans configuration relative to one another and wherein

n denotes 0, 1 or 2 and

W denotes a radical which can be cleaved off by hydrogenolysis or by acid, and subsequently splitting off the radical W and, if appropriate, the radical $R_2$ to form the free carboxyl groups, or

b) for the preparation of compounds of the formula I, in which Y and Z together denote oxygen, comprises

$b_1$) reacting a compound of the formula IV

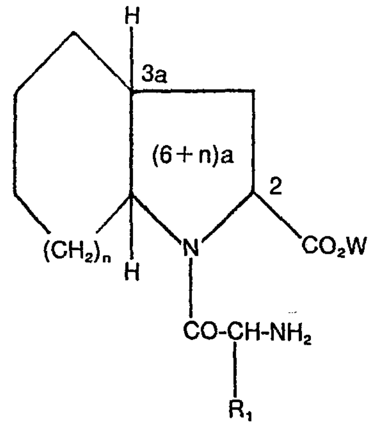 IV

in which the H atoms on the C atoms 3a and (6+n)a have the trans configuration relative to one another and wherein n and $R_1$ have the meanings as in formula I and W has the meaning as in formula III, with a compound of the formula V,

$R_2O_2C–CH=CH–CO–X$ V

wherein $R_2$ and X have the meanings as in formula I, and subsequently splitting off the radical W and, if appropriate, also the radical $R_2$ to form the free carboxyl groups, or,

$b_2$) reacting a compound of the formula IV mentioned under $b_1$) with a compound of the general formula VI, wherein $R_2$ has the meaning as in formula I, and with a compound of the general formula VII

| | |
|---|---|
| $OHC–CO_2R_2$ | $X–CO–CH_3$ |
| VI | VII |

wherein X has the meaning as in formula I, subsequently splitting off the radical W and, if appropriate, the radical $R_2$ to form the free carboxyl group, or

c) for the preparation of compounds of the formula I, in which Y and Z each denote hydrogen, comprises

$c_1$) reacting a compound of the formula IV mentioned under $b_1$) with a compound of the formula VIII

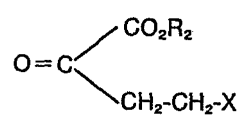 VIII

wherein $R_2$ and X have the meanings as in formula I, reducing the Schiff's bases obtained and subsequently splitting off the radical W and, if appropriate, the radical $R_2$ to form the free carboxyl groups, or

$c_2$) catalytically reducing a compound of the formula I in which Y and Z together denote oxygen, with hydrogen, or

d) for the preparation of compounds of the formula I, in which Y denotes hydroxyl and Z denotes hydrogen, comprises reducing a compound of the formula I, in which Y and Z together denote oxygen, catalytically with hydrogen or with a reducing agent, such as sodium borohydride,
and, if appropriate, converting the compounds obtained according to (a)–(d) into their physiologically acceptable salts.

7. A compound as claimed in any of claims 1 to 5 for the use as a medicine.

8. A compound as claimed in claim 7 for the use as a medicine for treating high blood pressure.

9. A compound as claimed in claim 7 or 8 for the use in combination with a diuretic.

10. A compound of the formula III',

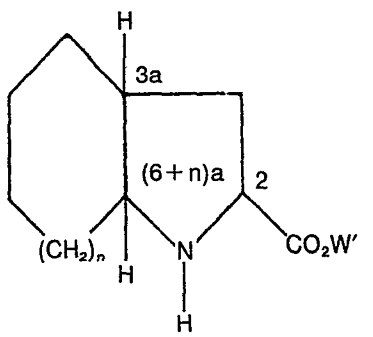

III'

in which the H atom on the C atoms 3a and (6+n)a have the trans configuration relative to one another and in which

n denotes 0, 1 or 2,

W' denotes hydrogen, $(C_1–C_{18})$-alkyl or $(C_7–C_{10})$-aralkyl.

11. A process for the preparation of the compounds of the formula III' as claimed in claim 10 which comprises

a) acylating a compound of the formula IX

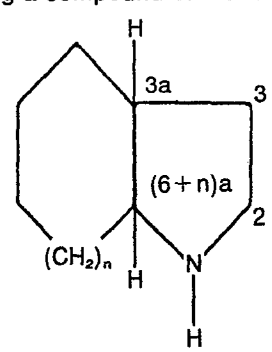

IX

wherein the H atom on the C atoms 3a and (6+n)a have the trans configuration relative to one another and wherein n denotes the number 0, 1 or 2, subsequently anodically oxidizing the compound obtained with an aliphatic alcohol in the presence of a conducting salt to give a compound of the formula X

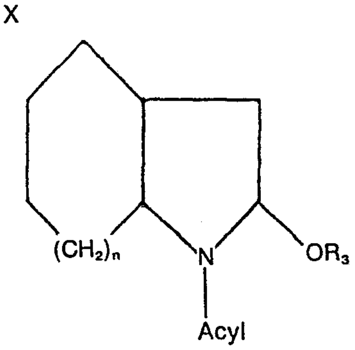

X

wherein n has the above-mentioned meaning, Acyl is an aliphatic or aromatic acyl radical and $R_3$ denotes $(C_1–C_4)$-alkyl, reacting this product with trimethylsilyl cyanide in an aprotic solvent in the presence of a Lewis acid to give a compound of the formula XI

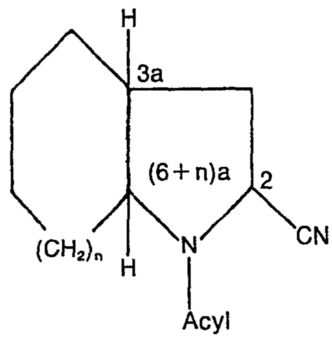

XI

wherein the H atoms on the C atoms 3a and (6+n)a have the trans configuration relative to one another and wherein n has the above-mentioned meaning and hydrolyzing the latter by the action of acids or bases to give a compound of the formula III' with W' = hydrogen and if appropriate, esterifying the latter, or

b) reacting a compound of the formula XII

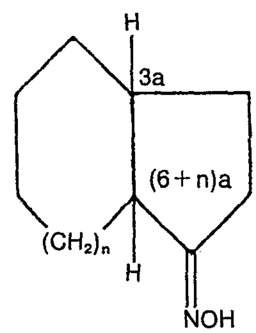

XII

wherein the H atoms on the C atoms 3a and (6+n)a have the trans configuration and n has the above-mentioned meaning, in a Beckmann rearrangement to give a compound of the formula XIII

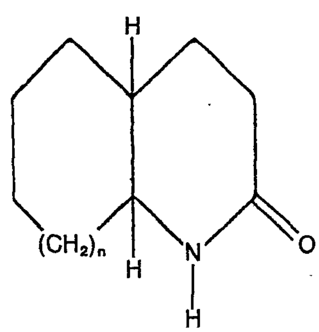

XIII

wherein n has the above-mentioned meaning, halogenating the latter to give a compound of the formula XIV

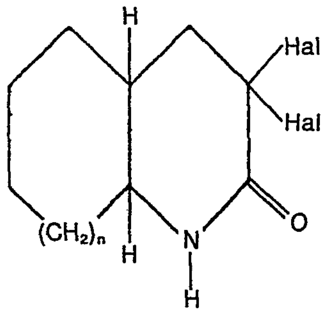

XIV

wherein n has the above-mentioned meaning and Hal denotes a halogen atom, catalytically reducing the latter in a polar protic solvent with addition of an acid acceptor to give a compound of the formula XV

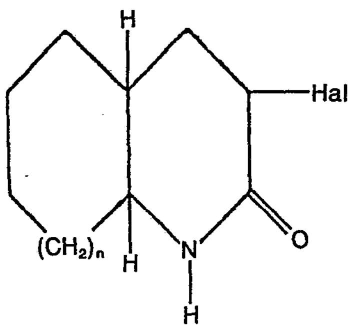

XV

wherein n and Hal have the above-mentioned meanings, and subsequently reacting the latter in an alcoholic solvent under the action of a base to give a compound of the formula III′ with W′ = hydrogen and optionally esterifying the latter, or

c) reducing a compound of the formula XVI

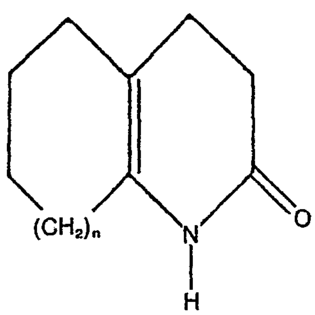

XVI

wherein n has the above-mentioned meaning, with sodium formate and formic acid to give a compound of the formula XIII mentioned under b), and further reacting the latter as described under b), or

d) converting compounds of the formula XXIa or b, in which n has the previous meaning,

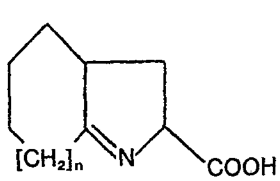

XXIa

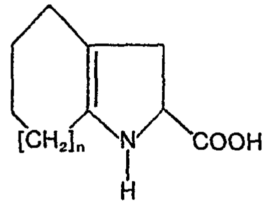

XXIb

optionally after conversion into their $C_1$–$C_{18}$-alkyl or $C_7$–$C_{10}$-aralkyl esters, by catalytic hydrogenation in the presence of transition metal catalysts or by reduction with borane-amine complexes or complex borohydrides, into compounds of the formula III′, in which W′ represents hydrogen, alkyl having 1 to 18 C atoms or aralkyl having 7 to 10 C atoms, saponifying, if appropriate, esters of the formula III′ and, if W′ is hydrogen, optionally esterifying to give compounds of the formula III′, in which n has the previous meaning and W′ represents alkyl having 1 to 18 C atoms or aralkyl having 7 to 10 C atoms.

12. A pharmaceutical composition containing a compound as claimed in any of claims 1 to 5.

13. A composition as claimed in claim 12, characterized in that it additionally contains a diuretic.

**Claims for the Contracting state: Austria**

1. A process for the preparation of the compounds of the formula I

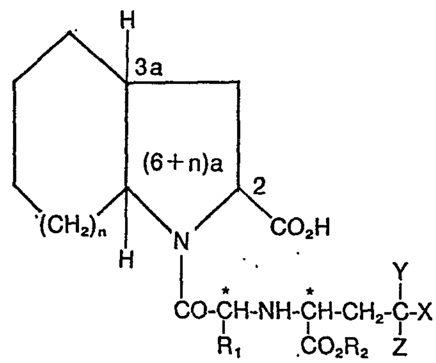

I

in which the hydrogen atoms on the bridgehead C atoms 3a and (6+n)a have the trans configuration relative to one another and wherein

n denotes 0, 1 or 2,
$R_1$ denotes methyl,
$R_2$ denotes hydrogen, ($C_1$–$C_4$)-alkyl or benzyl,
Y denotes hydrogen or hydroxyl,
Z denotes hydrogen or
Y and Z together denote oxygen and
X denotes phenyl,

and its physiologically acceptable salts, which

a) comprises reacting a compound of the formula II

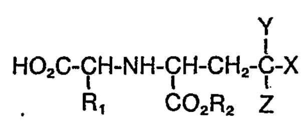

II

wherein $R_1$, $R_2$, X, Y and Z have the meanings as in formula I, with a compound of the formula III

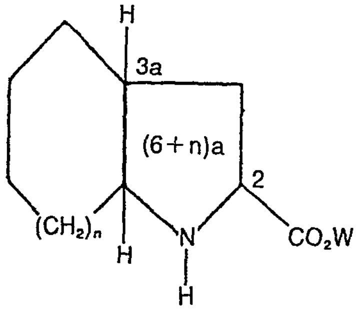

III

in which the H atoms on the C atoms 3a and (6+n)a have the trans configuration relative to one another and wherein

n denotes 0, 1 or 2 and

W denotes a radical which can be cleaved off by hydrogenolysis or by acid, and subsequently splitting off the radical W and, if appropriate, the radical $R_2$ to form the free carboxyl groups, or

b) for the preparation of compounds of the formula I, in which Y and Z together denote oxygen, comprises

$b_1$) reacting a compound of the formula IV

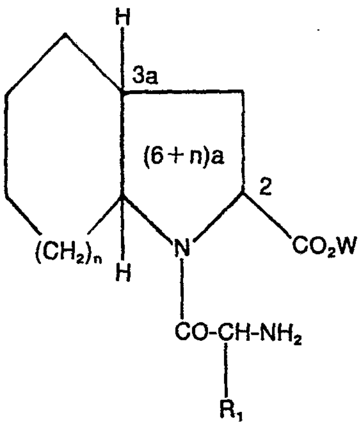

IV

in which the H atoms on the C atoms 3a and (6+n)a have the trans configuration relative to one another and wherein n and $R_1$ have the meanings as in formula I and W has the meaning as in formula III, with a compound of the formula V,

$$R_2O_2C\text{–}CH=CH\text{–}CO\text{–}X \qquad V$$

wherein $R_2$ and X have the meanings as in formula I, and subsequently splitting off the radical W and, if appropriate, also the radical $R_2$ to form the free carboxyl groups, or,

$b_2$) reacting a compound of the formula IV mentioned under $b_1$) with a compound of the general formula VI, wherein $R_2$ has the meaning as in formula I, and with a compound of the general formula VII

$OHC\text{–}CO_2R_2$ $\qquad$ $X\text{–}CO\text{–}CH_3$

VI $\qquad$ VII

wherein X has the meaning as in formula I, subsequently splitting off the radical W and, if appropriate, the radical $R_2$ to form the free carboxyl group, or

c) for the preparation of compounds of the formula I, in which Y and Z each denote hydrogen, comprises

$c_1$) reacting a compound of the formula IV mentioned under $b_1$) with a compound of the formula VIII

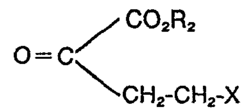

VIII

wherein $R_2$ and X have the meanings as in formula I, reducing the Schiff's bases obtained and subsequently splitting off the radical W and, if appropriate, the radical $R_2$ to form the free carboxyl groups, or

$c_2$) catalytically reducing a compound of the formula I in which Y and Z together denote oxygen, with hydrogen, or

d) for the preparation of compounds of the formula I, in which Y denotes hydroxyl and Z denotes hydrogen, comprises reducing a compound of the formula I, in which Y and Z together denote oxygen, catalytically with hydrogen or with a reducing agent, such as sodium borohydride,

and, if appropriate, converting the compounds obtained according to (a)–(d) into their physiologically acceptable salts.

2. A process for the preparation of compounds of the formula I as claimed in claim 1, characterized in that the C atom in position 2 of the bicyclic ring system and the C atom in the side chain labeled with an asterisk have the S configuration.

3. A process for the preparation of compounds as claimed in claim 1 or 2, characterized in that $R_2$ denotes hydrogen or ethyl, and its physiologically acceptable salts.

4. A process as claimed in any of claims 1 to 3, characterized in that

N-(1-S-carboethoxy-3-phenyl-propyl)-S-alanyl-2S,3aR,7aS-octahydroindole-2-carboxylic acid or its physiologically acceptable salt is prepared.

5. A process as claimed in any of claims 1 to 3, characterized in that

N-(1-S-carboethoxy-3-phenyl-propyl)-S-alanyl-2S,3aS,7aR-octahydroindole-2-carboxylic acid or and its physiologically acceptable salt is prepared.

6. A process for the preparation of compounds of the formula III',

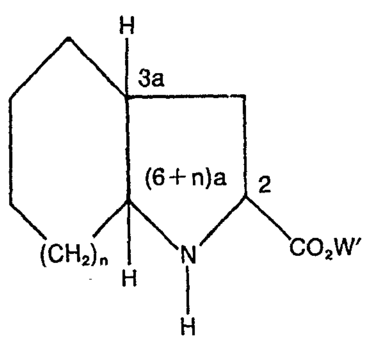

III'

in which the H atom on the C atoms 3a and (6+n)a have the trans configuration relative to one another and in which

n denotes 0, 1 or 2,

W' denotes hydrogen, $(C_1-C_{18})$-alkyl or $(C_7-C_{10})$-aralkyl, which comprises

a) acylating a compound of the formula IX

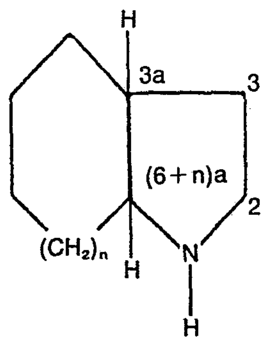

IX

wherein the H atom on the C atoms 3a and (6+n)a have the trans configuration relative to one another and wherein n denotes the number 0, 1 or 2, subsequently anodically oxidizing the compound obtained with an aliphatic alcohol in the presence of a conducting salt to give a compound of the formula X

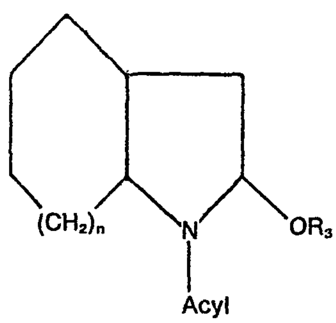

X

wherein n has the above-mentioned meaning, Acyl is an aliphatic or aromatic acyl radical and $R_3$ denotes $(C_1-C_4)$-alkyl, reacting this product with trimethylsilyl cyanide in an aprotic solvent in the presence of a Lewis acid to give a compound of the formula XI

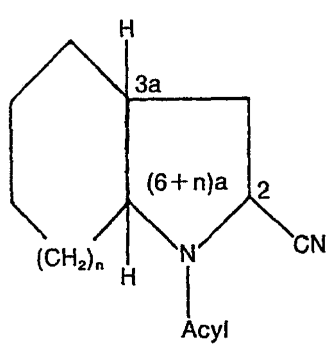

XI

wherein the H atoms on the C atoms 3a and (6+n)a have the trans configuration relative to one another and wherein n has the above-mentioned meaning and hydrolyzing the latter by the action of acids or bases to give a compound of the formula III' with W' = hydrogen and if appropriate, esterifying the latter, or

b) reacting a compound of the formula XII

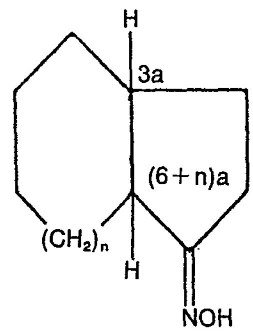

XII

wherein the H atoms on the C atoms 3a and (6+n)a have the trans configuration and n has the above-mentioned meaning, in a Beckmann rearrangement to give a compound of the formula XIII

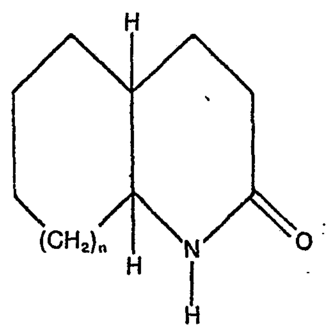

XIII

wherein n has the above-mentioned meaning, halogenating the latter to give a compound of the formula XIV

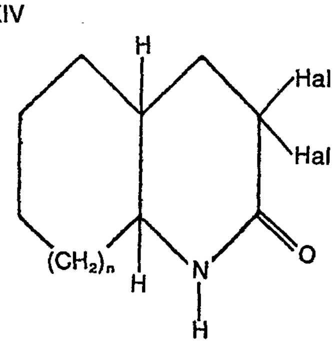

XIV

wherein n has the above-mentioned meaning and Hal denotes a halogen atom, catalytically reducing the latter in a polar protic solvent with addition of an acid acceptor to give a compound of the formula XV

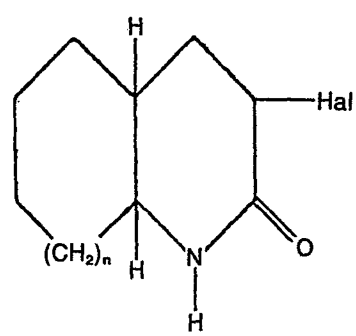

XV

wherein n and Hal have the above-mentioned meanings, and subsequently reacting the latter in an alcoholic solvent under the action of a base to give a compound of the formula III′ with W′ = hydrogen and optionally esterifying the latter, or

c) reducing a compound of the formula XVI

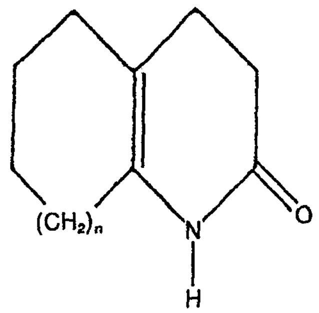

XVI

wherein n has the above-mentioned meaning, with sodium formate and formic acid to give a compound of the formula XIII mentioned under b), and further reacting the latter as described under b), or

d) converting compounds of the formula XXIa or b, in which n has the previous meaning,

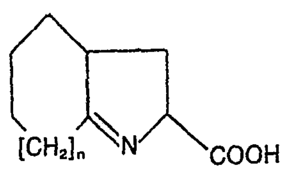

XXIa

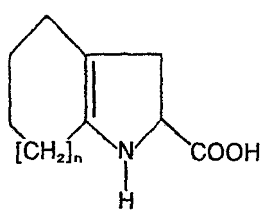

XXIb

optionally after conversion into their $C_1$–$C_{18}$-alkyl or $C_7$–$C_{10}$-aralkyl esters, by catalytic hydrogenation in the presence of transition metal catalysts or by reduction with borane-amine complexes or complex borohydrides, into compounds of the formula III′, in which W′ represents hydrogen, alkyl having 1 to 18 C atoms or aralkyl having 7 to 10 C atoms, saponifying, if appropriate, esters of the formula III′ and, if W′ is hydrogen, optionally esterifying to give compounds of the formula III′, in which n has the previous meaning and W′ represents alkyl having 1 to 18 C atoms or aralkyl having 7 to 10 C atoms.

7. A process for the preparation of a pharmaceutical composition containing a compound of the formula I as claimed in any of claims 1 to 5, characterized in that this compound is brought in a suitable form for administration.

8. A process as claimed in claim 7, characterized in that a diuretic is added.

**Revendications pour les Etats contractants: BE, CH, DE, FR, GB, IT, Li, Lu, NL, SE**

1. Composé de formule I

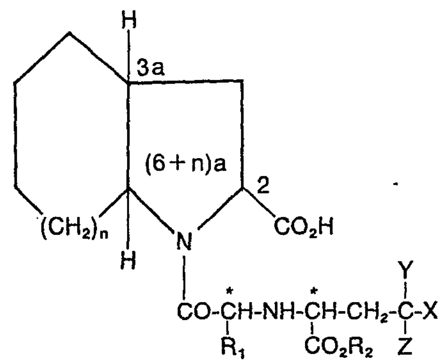

I

dans laquelle les atomes d'hydrogène sur les atomes de carbone de tête de pont 3a et (6 + n)a sont en configuration trans l'un par rapport à l'autre, et dans laquelle

n = 0, 1 ou 2,

$R_1$ représente le groupe méthyle,

$R_2$ représente un atome d'hydrogène, un groupe alkyle en $C_1$–$C_4$ ou benzyle,

Y représente un atome d'hydrogène ou le groupe hydroxy,

Z représente un atome d'hydrogène, ou

Y et Z représentent ensemble un atome d'oxygène, et

X représente le groupe phényle,

et les sels physiologiquement acceptables de celui-ci.

2. Composé de formule I selon la revendication 1, caractérisé en ce que l'atome de carbone en position 2 du système bicyclique, ainsi que les atomes de carbone de la chaîne latérale qui sont marqués par un astérisque, présentent la configuration S, et les sels physiologiquement acceptables de celui-ci.

3. Composé de formule I selon la revendication 1 ou 2, caractérisé en ce que $R_2$ représente un atome d'hydrogène ou le groupe éthyle, et les sels physiologiquement acceptables de celui-ci.

4. Acide N-(1-S-carbéthoxy-3-phényl-propyl)-S-alanyl-2S,3aR,7aS-octahydro-indole-2-carboxylique, et les sels physiologiquement acceptables de celui-ci.

5. Acide N-(1-S-carbéthoxy-3-phényl-propyl)-S-alanyl-2S,3aS,7aR-octahydro-indole-2-carboxylique, et les sels physiologiquement acceptables de celui-ci.

6. Procédé pour la préparation des composés de formule I selon une des revendications 1 à 5, caractérisé en ce que

a) on fait réagir un composé de formule II

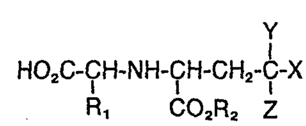

II

dans laquelle $R_1$, $R_2$, X, Y et Z ont les mêmes significations que dans la formule I, avec un composé de formule III

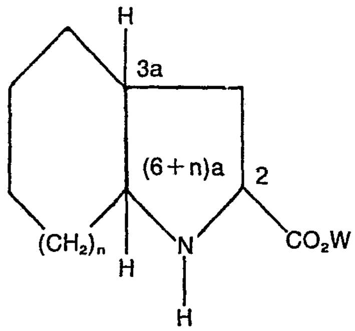

III

dans laquelle les atomes d'hydrogène sur les atomes de carbone 3a et (6+n)a sont en configuration trans l'un par rapport à l'autre,

n = 0, 1 ou 2, et

W représente un reste séparable par hydrogénolyse ou par voie acide,

et on sépare ensuite le reste W et, éventuellement, le reste $R_2$ pour obtenir les groupes carboxy libres, ou

b) on fait réagir, pour la préparation des composés de formule I dans lesquels Y et Z représentent ensemble un atome d'oxygène,

$b_1$) un composé de formule IV

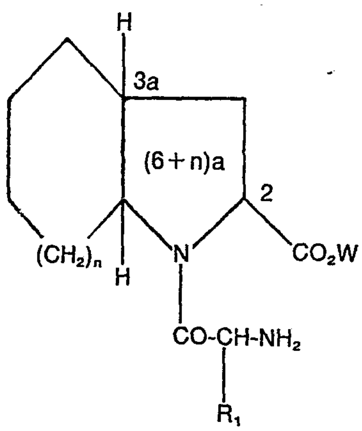

IV

dans laquelle les atomes d'hydrogène sur les atomes de carbone 3a et (6+n)a sont en configuration trans l'un par rapport à l'autre, et dans laquelle n et $R_1$ ont les mêmes significations que dans la formule I et W a la même signification que dans la formule III, avec un composé de formule V

$R_2O_2C–CH=CH–CO–X$ V

dans laquelle $R_2$ et X ont les mêmes significations que dans la formule I, et on sépare ensuite le reste W et, éventuellement, aussi le reste $R_2$, pour former les groupes carboxy libres, ou

$b_2$) un composé de formule IV indiquée en $b_1$) avec un composé de formule générale VI

$OHC–CO_2R_2$ VI

dans laquelle $R_2$ a la même signification que dans la formule I, et avec un composé de formule générale VII

$X–CO–CH_3$ VII

dans laquelle X a la même signification que dans la formule I, et on sépare ensuite le reste W et éventuellement le reste $R_2$, en formant les groupes carboxy libres, ou

c) pour la préparation des composés de formule I dans lesquels Y et Z représentent chacun un atome d'hydrogène,

$c_1$) on fait réagir un composé de formule IV indiquée en $b_1$) avec un composé de formule VIII

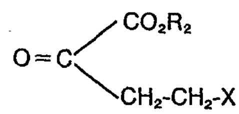

VIII

dans laquelle $R_2$ et X ont les mêmes significations que dans la formule I, on réduit les bases de Schiff obtenues et on sépare ensuite le reste W et éventuellement le reste $R_2$ en formant les groupes carboxy libres, ou

$c_2$) on réduit avec de l'hydrogène, par voie catalytique, un composé de formule I dans lequel Y et Z représentent ensemble un atome d'oxygène, ou

d) pour la préparation des composés de formule I dans lesquels Y représente un groupe hydroxy et Z représente un atome d'hydrogène, on réduit au moyen d'hydrogène, par voie catalytique, ou avec un réducteur tel que le borohydrure de sodium, un composé de formule I dans lequel X et Y représentent ensemble un atome d'oxygène,

et on transforme éventuellement les composés obtenus selon (a)–(d) en leurs sels physiologiquement acceptables.

7. Composé selon l'une des revendications 1 à 5, pour utilisation comme médicament.

8. Composé selon la revendication 7, pour utilisation comme médicament dans le traitement de l'hypertension.

9. Composé selon la revendication 7 ou 8, pour utilisation en association avec un diurétique.

10. Composé de formule III'

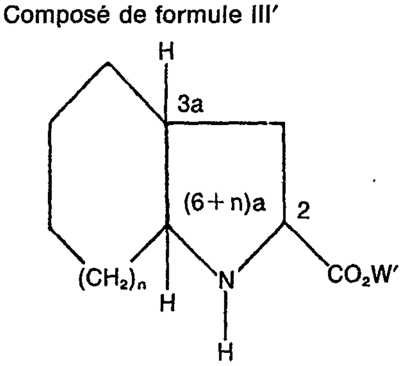

III'

dans laquelle les atomes d'hydrogène sur les atomes de carbone 3a et (6+n)a sont en configuration trans l'un par rapport à l'autre, et dans laquelle

n = 0, 1 ou 2,

W' représente un atome d'hydrogène, un groupe alkyle en $C_1$–$C_{18}$ ou un groupe aralkyle en $C_7$–$C_{10}$.

11. Procédé pour la préparation des composés de formule III' selon la revendication 10, caractérisé en ce que

a) on soumet à une acylation un composé de formule IX

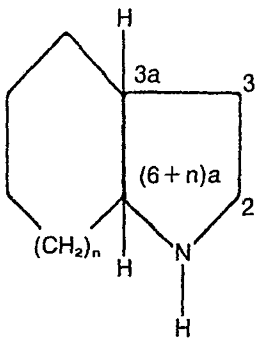

IX

dans laquelle les atomes d'hydrogène sur les atomes de carbone 3a et (6+n)a sont en configuration trans l'un par rapport à l'autre, et dans laquelle n représente 0, 1 ou 2, on le soumet ensuite à une oxydation anodique avec un alcool aliphatique, en présence d'un sel conducteur, pour obtenir un composé de formule X

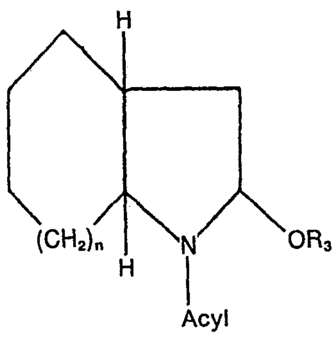

X

dans laquelle n a la signification donnée plus haut, «acyle» étant un radical acyle aliphatique ou aromatique, et $R_3$ représente un groupe alkyle en $C_1$–$C_4$, on fait réagir ce composé avec du cyanure de triméthylsilyle dans un solvant organique aprotique, en présence d'un acide de Lewis, pour aboutir à un composé de formule XI

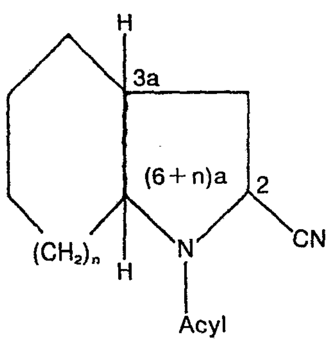

XI

dans laquelle les atomes d'hydrogène sur les atomes de carbone 3a et (6+n)a sont en configuration trans l'un par rapport à l'autre, et dans laquelle n a la signification donnée plus haut, puis on hydrolyse celui-ci sous l'action d'acides ou de bases, pour aboutir à un composé de formule III' dans lequel W' est un atome d'hydrogène, et on estérifie éventuellement ce dernier, ou

b) on fait réagir un composé de formule XII

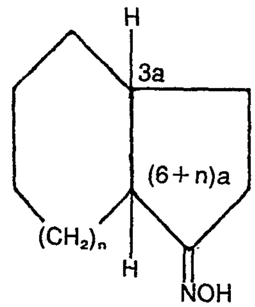

XII

dans laquelle les atomes d'hydrogène sur les atomes de carbone 3a et (6+n)a sont en configuration trans et n a la signification donnée plus haut, dans une transposition de Beckmann, pour aboutir à un composé de formule XIII

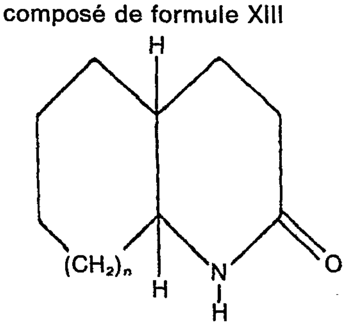

XIII

dans laquelle n a la signification donnée plus haut, on soumet celui-ci à une halogénation pour aboutir à un composé de formule XIV,

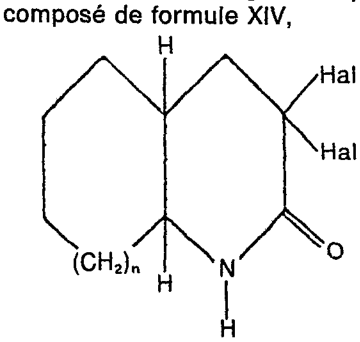

XIV

dans laquelle n a la signification donnée plus haut et Hal représente un atome d'halogène, on réduit ce composé par voie catalytique, dans un solvant polaire protique, en utilisant un accepteur d'acide, pour aboutir à un composé de formule XV

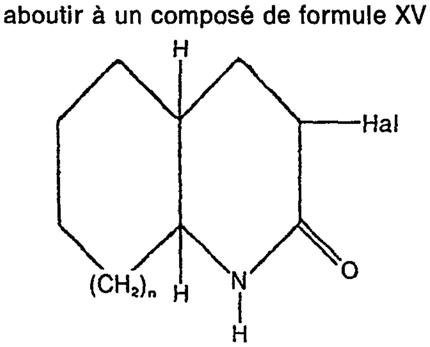

XV

dans laquelle n et Hal ont les significations données plus haut, et on transforme ensuite celui-ci, sous l'effet d'une base, dans un solvant alcoolique, en un composé de formule III' dans lequel W' est un atome d'hydrogène, puis on estérifie éventuellement celui-ci, ou

c) on réduit avec du formiate de sodium et de l'acide formique un composé de formule XVI

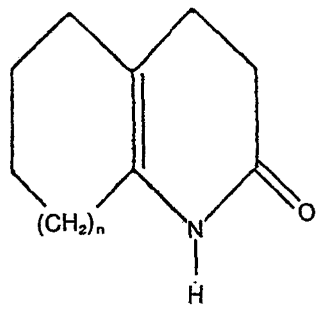

XVI

dans laquelle n a la signification donnée plus haut, pour aboutir à un composé de formule XIII indiquée en b), et on poursuit la transformation de celui-ci comme décrit en b), ou

d) on transforme des composés de formule XXIa ou b

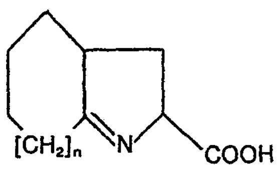

XXIa

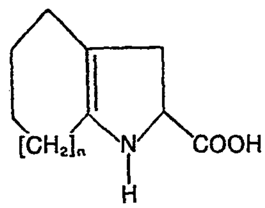

XXIb

dans lesquelles n a la signification donnée plus haut, éventuellement après conversion en leurs esters d'alkyle en $C_1$–$C_{18}$ ou d'aralkyle en $C_7$–$C_{10}$, par hydrogénation catalytique en présence de catalyseurs à base de métaux de transition, ou par réduction avec des complexes borane-amine ou des borohydrures complexes, en composés de formule III' dans lesquels W' représente un atome d'hydrogène, un groupe alkyle ayant de 1 à 18 atomes de carbone ou un groupe aralkyle ayant de 7 à 10 atomes de carbone, on saponifie éventuellement les esters de formule III' obtenus et, au cas où W' est un atome d'hydrogène, on les estérifie éventuellement, pour aboutir à des composés de formule III' dans lesquels W' représente un groupe alkyle ayant de 1 à 18 atomes de carbone ou un groupe aralkyle ayant de 7 à 10 atomes de carbone.

12. Produit pharmaceutique contenant un composé selon l'une des revendications 1 à 5.

13. Produit selon la revendication 12, caractérisé en ce qu'il contient en outre un diurétique.

**Revendications pour l'Etat contractant: AT**

1. Procédé pour la préparation des composés de formule I

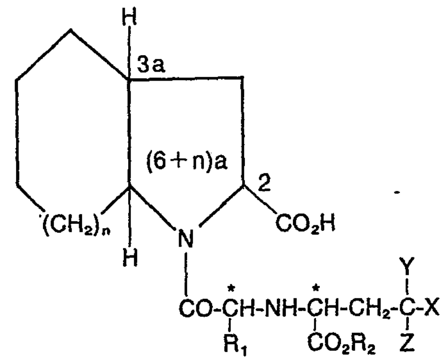

I

dans laquelle les atomes d'hydrogène sur les atomes de carbone de tête de pont 3a et (6+n)a sont en configuration trans l'un par rapport à l'autre, et dans laquelle

n = 0, 1 ou 2,

$R_1$ représente le groupe méthyle,

$R_2$ représente un atome d'hydrogène, un groupe alkyle en $C_1$–$C_4$ ou benzyle,

Y représente un atome d'hydrogène ou le groupe hydroxy,

Z représente un atome d'hydrogène, ou

Y et Z représentent ensemble un atome d'oxygène, et

X représente le groupe phényle,

et des sels physiologiquement acceptables de ceux-ci, caractérisé en ce que

a) on fait réagir un composé de formule II

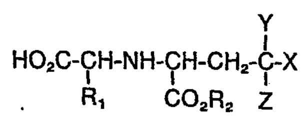

II

dans laquelle $R_1$, $R_2$, X, Y et Z ont les mêmes significations que dans la formule I, avec un composé de formule III

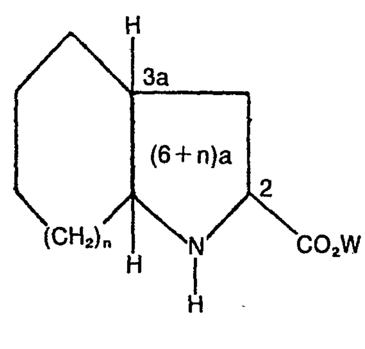

III

dans laquelle les atomes d'hydrogène sur les atomes de carbone 3a et (6+n)a sont en configuration trans l'un par rapport à l'autre,

n = 0, 1 ou 2, et

W représente un reste séparable par hydrogénolyse ou par voie acide,

et on sépare ensuite le reste W et, éventuellement, le reste $R_2$ pour obtenir les groupes carboxy libres, ou

b) on fait réagir, pour la préparation des composés de formule I dans lesquels Y et Z représentent ensemble un atome d'oxygène,

$b_1$) un composé de formule IV

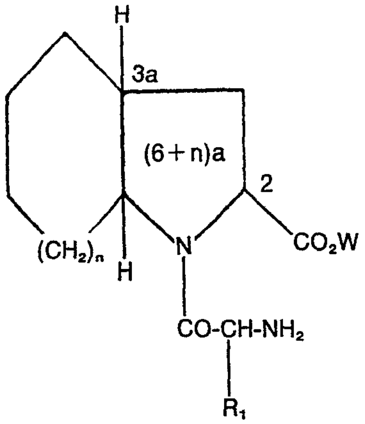

IV

dans laquelle les atomes d'hydrogène sur les atomes de carbone 3a et (6+n)a sont en configuration trans l'un par rapport à l'autre, et dans laquelle n et $R_1$ ont les mêmes significations que dans la formule I et W a la même signification que dans la formule III, avec un composé de formule V

$$R_2O_2C\text{–}CH=CH\text{–}CO\text{–}X \qquad V$$

dans laquelle $R_2$ et X ont les mêmes significations que dans la formule I, et on sépare ensuite le reste W et, éventuellement, aussi le reste $R_2$, pour former les groupes carboxy libres, ou

$b_2$) un composé de formule IV indiquée en $b_1$) avec un composé de formule générale VI

$$OHC\text{–}CO_2R_2 \qquad VI$$

dans laquelle $R_2$ a la même signification que dans la formule I, et avec un composé de formule générale VII

$$X\text{–}CO\text{–}CH_3 \qquad VII$$

dans laquelle X a la même signification que dans la formule I, et on sépare ensuite le reste W et éventuellement le reste $R_2$, en formant les groupes carboxy libres, ou

c) pour la préparation des composés de formule I dans lesquels Y et Z représentent chacun un atome d'hydrogène,

$c_1$) on fait réagir un composé de formule IV indiquée en $b_1$) avec un composé de formule VIII

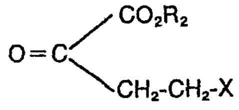

VIII

dans laquelle $R_2$ et X ont les mêmes significations que dans la formule I, on réduit les bases de Schiff obtenues et on sépare ensuite le reste W et éventuellement le reste $R_2$ en formant les groupes carboxy libres, ou

$c_2$) on réduit avec de l'hydrogène, par voie catalytique, un composé de formule I dans lequel Y et Z représentent ensemble un atome d'oxygène, ou

d) pour la préparation des composés de formule I dans lesquels Y représente un groupe hydroxy et Z représente un atome d'hydrogène, on réduit au moyen d'hydrogène, par voie catalytique, ou avec un réducteur tel que le borohydrure de sodium, un composé de formule I dans lequel X et Y représentent ensemble un atome d'oxygène, et on transforme éventuellement les composés obtenus selon (a)–(d) en leurs sels physiologiquement acceptables.

2. Procédé pour la préparation de composés de formule I selon la revendication 1, caractérisé en ce que l'atome de carbone en position 2 du système bicyclique, ainsi que les atomes de carbone de la chaîne latérale qui sont marqués par un astérisque, présentent la configuration S.

3. Procédé pour la préparation de composés de formule I selon la revendication 1 ou 2, caractérisé en ce que $R_2$ représente un atome d'hydrogène ou le groupe éthyle.

4. Procédé selon l'une des revendications 1 à 3, caractérisé en ce que l'on prépare l'acide N-(1-S-carbéthoxy-3-phényl-propyl)-S-alanyl-2S,3aR,7aS-octahydro-indole-2-carboxylique ou un sel physiologiquement acceptable de celui-ci.

5. Procédé selon l'une des revendications 1 à 3, caractérisé en ce que l'on prépare l'acide N-(1-S-carbéthoxy-3-phényl-propyl)-S-alanyl-2S,3aS,7aR-octahydro-indole-2-carboxylique ou un sel physiologiquement acceptable de celui-ci.

6. Procédé pour la préparation des composés de formule III'

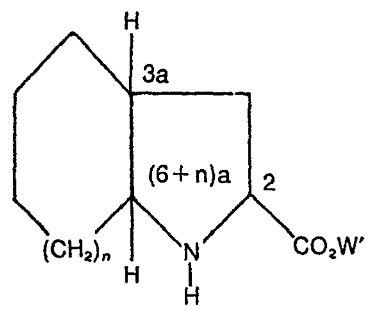

III'

dans laquelle les atomes d'hydrogène sur les atomes de carbone 3a et (6+n)a sont en configuration trans l'un par rapport à l'autre, et dans laquelle

n = 0, 1 ou 2,

W' représente un atome d'hydrogène, un groupe alkyle en $C_1$–$C_{18}$ ou un groupe aralkyle en $C_7$–$C_{10}$,

caractérisé en ce que

a) on soumet à une acylation un composé de formule IX

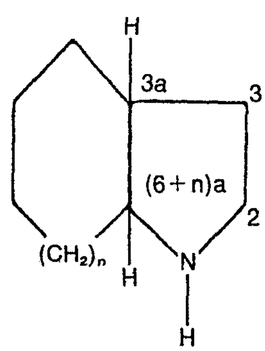

IX

dans laquelle les atomes d'hydrogène sur les atomes de carbone 3a et (6+n)a sont en configuration trans l'un par rapport à l'autre, et dans laquelle n représente 0, 1 ou 2, on le soumet ensuite à une oxydation anodique avec un alcool aliphatique, en présence d'un sel conducteur, pour obtenir un composé de formule X

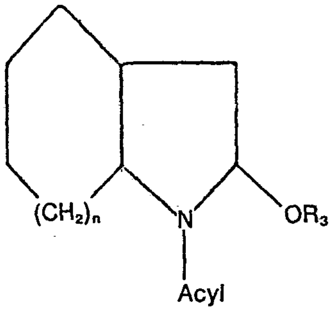

X

dans laquelle n a la signification donnée plus haut, acyle étant un radical acyle aliphatique ou aromatique, et $R_3$ représente un groupe alkyle en $C_1$–$C_4$, on fait réagir ce composé avec du cyanure de triméthylsilyle dans un solvant organique aprotique, en présence d'un acide de Lewis, pour aboutir à un composé de formule XI

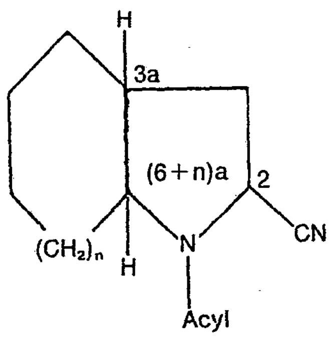

XI

dans laquelle les atomes d'hydrogène sur les atomes de carbone 3a et (6+n)a sont en configuration trans l'un par rapport à l'autre, et dans laquelle n a la signification donnée plus haut, puis on hydrolyse celui-ci sous l'action d'acides ou de bases, pour aboutir à un composé de formule III' dans lequel W' est un atome d'hydrogène, et on estérifie éventuellement ce dernier, ou

b) on fait réagir un composé de formule XII

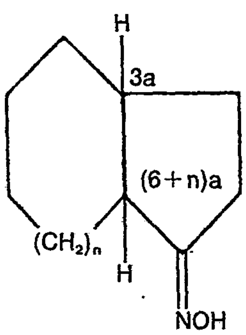

XII

dans laquelle les atomes d'hydrogène sur les atomes de carbone 3a et (6+n)a sont en configuration trans et n a la signification donnée plus haut, dans une transposition de Beckmann, pour aboutir à un composé de formule XIII

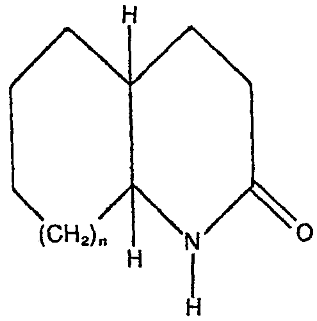

XIII

dans laquelle n a la signification donnée plus haut, on soumet celui-ci à une halogénation pour aboutir à un composé de formule XIV,

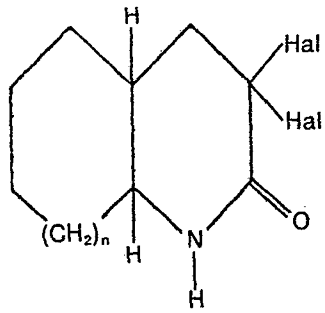

XIV

dans laquelle n a la signification donnée plus haut et Hal représente un atome d'halogène, on réduit ce composé par voie catalytique, dans un solvant polaire protique, en utilisant un accepteur d'acide, pour aboutir à un composé de formule XV

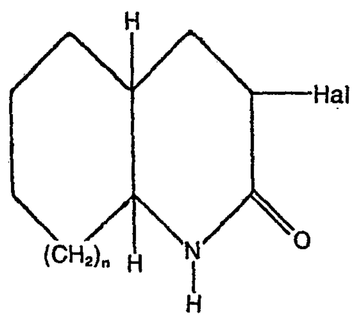

XV

dans laquelle n et Hal ont les significations données plus haut, et on transforme ensuite celui-ci, sous l'effet d'une base, dans un solvant alcoolique, en un composé de formule III' dans lequel W' est un atome d'hydrogène, puis on estérifie éventuellement celui-ci, ou

c) on réduit avec du formiate de sodium et de l'acide formique un composé de formule XVI

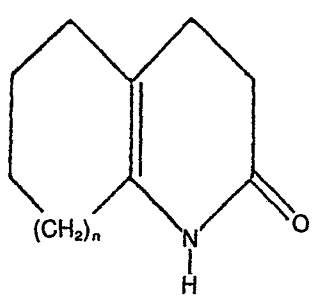

XVI

dans laquelle n a la signification donnée plus haut, pour aboutir à un composé de formule XIII indiquée en b), et on poursuit la transformation de celui-ci comme décrit en b), ou

d) on transforme des composés de formule XXIa ou b

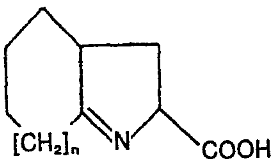

XXIa

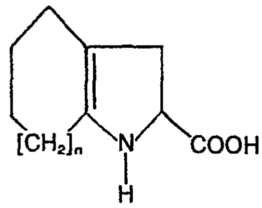

XXIb

dans lesquelles n a la signification donnée plus haut, éventuellement après conversion en leurs esters d'alkyle en $C_1$–$C_{18}$ ou d'aralkyle en $C_7$–$C_{10}$, par hydrogénation catalytique en présence de catalyseurs à base de métaux de transition, ou par réduction avec des complexes borane-amine ou des borohydrures complexes, en composés de formule III' dans lesquels W' représente un atome d'hydrogène, un groupe alkyle ayant de 1 à 18 atomes de carbone ou un groupe aralkyle ayant de 7 à 10 atomes de carbone, on saponifie éventuellement les esters de formule III' obtenus et, au cas où W' est un atome d'hydrogène, on les estérifie éventuellement, pour aboutir à des composés de formule III' dans lesquels W' représente un groupe alkyle ayant de 1 à 18 atomes de carbone ou un groupe aralkyle ayant de 7 à 10 atomes de carbone.

7. Procédé pour la préparation d'un produit pharmaceutique contenant un composé de formule I selon une des revendications 1 à 5, caractérisé en ce que l'on met celui-ci sous une forme d'administration appropriée.

8. Procédé selon la revendication 7, caractérisé en ce que l'on ajoute un diurétique.